# EUROPEAN PATENT SPECIFICATION

(11) **EP 3 638 643 B1**
(45) Date of publication and mention of the grant of the patent: **18.01.2023**
(21) Application number: 18729133.1
(22) Date of filing: 11.06.2018
(51) Int. Cl.: C07C 19/08, C07C 17/263, A61P 17/00, A61P 27/00

(54) **PROCESS FOR THE PREPARATION OF SEMIFLUORINATED ALKANES USING GRIGNARD REAGENTS**
VERFAHREN ZUR HERSTELLUNG VON SEMIFLUORIERTEN ALKANEN UNTER VERWENDUNG VON GRIGNARD-REAGENZIEN
PROCÉDÉ DE PRÉPARATION D'ALCANES SEMIFLUORÉS AU MOYEN DE RÉACTIFS DE GRIGNARD

(30) Priority: 12.06.2017 EP 17175493
(43) Date of publication of application: 22.04.2020
(73) Proprietor: Novaliq GmbH, 69120 Heidelberg (DE)
(72) Inventor: IVANOV BICHOVSKI, Plamen, 69221 Dossenheim (DE); LÖSCHER, Frank, 69198 Schriesheim (DE); NICOLETTI, Salvatore, 69124 Heidelberg (DE)
(74) Representative: Pharma Patents International AG
(86) International application number: PCT/EP2018/065312
(87) International publication number: WO 2018/228975

(56) References cited:
- CN-A- 106 316 777
- DE-A1- 19 536 504
- US-A1- 2013 084 250
- US-B1- 6 211 248
- SHIMIZU R ET AL: "Copper Catalyzed Grignard Cross-Coupling Reaction with beta-Perfluoroalkyl-Substituted Alkyl Halides", TETRAHEDRON LETTERS, ELSEVIER, AMSTERDAM, NL, vol. 37, no. 31, 29 July 1996 (1996-07-29) , pages 5557-5560, XP004029449, ISSN: 0040-4039, DOI: 10.1016/0040-4039(96)01155-0
- TERAO J ET AL: "Ni- or Cu-catalysed cross-coupling reaction of alkyl fluorides with Grignard reagents", JOURNAL OF THE AMERICAN CHEMICAL SOCIETY, AMERICAN CHEMICAL SOCIETY, US, vol. 125, no. 19, 22 April 2003 (2003-04-22), pages 5646-5647, XP002277410, ISSN: 0002-7863, DOI: 10.1021/JA034201P
- TAKANORI IWASAKI ET AL: "Co-Catalyzed Cross-Coupling of Alkyl Halides with Tertiary Alkyl Grignard Reagents Using a 1,3-Butadiene Additive", JOURNAL OF THE AMERICAN CHEMICAL SOCIETY, vol. 135, no. 26, 3 July 2013 (2013-07-03) , pages 9604-9607, XP055402611, US ISSN: 0002-7863, DOI: 10.1021/ja404285b cited in the application
- TERAO J ET AL: "Nickel-catalysed cross-coupling reaction of Grignard reagents with alkyl halides and tosylates: remarkable effect of 1,3-butadienes", JOURNAL OF THE AMERICAN CHEMICAL SOCIETY, AMERICAN CHEMICAL SOCIETY, US, vol. 124, no. 16, 28 March 2002 (2002-03-28), pages 4222-4223, XP002277408, ISSN: 0002-7863, DOI: 10.1021/JA025828V
- SINGH S P ET AL: "Cross-coupling of Grignard reagents with alkyl halides or tosylates by the use of nickel or palladium containing perovskite", TETRAHEDRON LETTERS, ELSEVIER, AMSTERDAM, NL, vol. 52, no. 7, 16 February 2011 (2011-02-16), pages 774-776, XP027597406, ISSN: 0040-4039, DOI: 10.1016/J.TETLET.2010.12.011 [retrieved on 2011-01-12]
- JUN TERAO ET AL: "Cross-Coupling Reaction of Alkyl Halides with Grignard Reagents Catalyzed by Ni, Pd, or Cu Complexes with [pi]-Carbon Ligand(s)", ACCOUNTS OF CHEMICAL RESEARCH., vol. 41, no. 11, 18 November 2008 (2008-11-18), pages 1545-1554, XP055402761, US ISSN: 0001-4842, DOI: 10.1021/ar800138a
- MARIE PIERRE KRAFFT ET AL: "Chemistry, Physical Chemistry, and Uses of Molecular Fluorocarbon-Hydrocarbon Diblocks, Triblocks, and Related Compounds - Unique "Apolar" Components for Self-Assembled Colloid and Interface Engineering", CHEMICAL REVIEWS, vol. 109, no. 5, 13 May 2009 (2009-05-13), pages 1714-1792, XP055402610, US ISSN: 0009-2665, DOI: 10.1021/cr800260k

## Description

### FIELD OF THE INVENTION

The present invention relates to a process for the synthesis of semifluorinated alkanes.

### BACKGROUND OF THE INVENTION

Semifluorinated alkanes are physically, chemically and physiologically inert compounds, which find application in medicine, in particular in the ophthalmic and dermatological field.

As described in Chem. Rev. 2009, 109, 1714-1792, a process commonly used for the production of semifluorinated alkanes such as, for example, semifluorinated alkanes of the type CₙF₂ₙ₊₁CₘH₂ₘ₊₁ is a process comprising the free radical addition of F-alkyl iodides, CₙF₂ₙ₊₁I, to a double bond of an alkene compound, followed by reductive dehalogenation of the resulting iodinated adduct. The addition of CₙF₂ₙ₊₁I to a terminal alkene is conducted according to the following scheme:

CₙF₂ₙ₊₁I + CH₂=CHCₘ₋₂H₂ₘ₋₃ → CₙF₂ₙ₊₁CH₂CHICₘ₋₂H₂ₘ₋₃

Subsequent reductive dehalogenation of the iodo intermediate CnF₂ₙ₊₁CH₂CHICₘ₋₂H₂ₘ₋₃ is commonly performed with zinc/HCl or on an industrial scale by catalytic hydrogenation utilizing palladium on charcoal. Thorough purification of the iodo intermediate and/or the dehalogenated crude products is indispensable, especially when physicochemical investigation or biomedical applications are intended. It is usually achieved by one or more subsequent distillations, due to the formation of isomeric and olefinic by-products with highly comparable physicochemical characteristics.

Similar to the linear semifluorinated alkanes, the synthesis of semifluorinated alkanes with branched hydrocarbon chains comprises the free radical addition of F-alkyl iodides CₙF₂ₙ₊₁I to a multiple bond, followed by reductive dehalogenation of the resulting iodinated adduct. An example of reductive dehalogenation of a semifluorinated alkaneiodinated compound with branched hydrocarbon chain has been reported as follows:

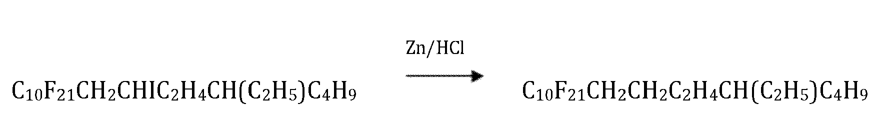

DE 392552 A1 discloses a process for the preparation of symmetric alpha, omega-bis-perfluoroalkyl alkanes by dimerization of perfluoroalkyl alkane bromides or iodides in the presence of magnesium chips. The yield of the dimerization products can be raised by addition of catalytic amounts of a perfluoroalkylalkene or by addition of a transition metal complex of group 6, 7 or 8 of the PSE.

Furthermore, DE 195 36 504 A1 discloses an alternative 2-step route to semifluorinated alkanes by reaction of a perfluoroalkylalkyl-halogenide with an alkene in the presence of azo-isobutyronitrile and subsequent reduction of the halogenated intermediate with zinc in the presence of acetic acid.

S. Lavaire et al. describe in Tetrahedron: Asymmetry 9 (1998) 213-226 the selectivity of the nucleophilic F-alkylation of carbonylated carbohydrates. Prior to the reaction with the carbonylated carbohydrates, the perfluoroalkyl Grignard reagent (C₄F₉MgBr or C₆F₁₃MgBr) was generated in situ by reaction of a perfluoroalkyl iodide with ethylmagnesium bromide at -45°C.

WO 2005/074593 A2 discloses coupling reactions of branched secondary alkyl iodides which are partially perfluorinated with allyl magnesium bromide to yield olefinic products with a branched fluorinated alkyl portion.

In WO 00/66489 A1 the reaction of 4-bromophenyl magnesium bromide with 1,1,1,2,2,3,3,4,4,5,5,6,6-tridecafluoro-8-iodooctane in the presence of CuCl to yield 1,1,1,2,2,3,3,4,4,5,5,6,6-tridecafluoro-8-(4-bromophenyl)-octane is described.

T. Iwasaki et al. describe in J. Am. Chem. Soc. 2013, 135, 9604-9607 the cobalt catalysed cross coupling of alkyl (pseudo)halides with alkyl Grignard reagent in the presence of 1,3 butadiene and LiI as an additive. Satisfactory yields of the cross-coupled products were only achieved using CoCl₂ as the transition metal chloride. In contrast to this, neither NiCl₂ nor PdCl₂ nor CuCl₂ afforded the cross-coupled product in a preparative useful yield when used as the catalyst.

Furthermore, T. Iwasaki et al. describe in Synthesis 2014, 46, 1583-1592 the same cobalt catalysed cross-coupling reaction under optimized conditions starting from primary, secondary or tertiary alkyl halides. The reaction is carried out in the presence of isoprene, CoCl₂ and LiI.

The preparation of semifluorinated alkane compounds via reaction of a non-fluorinated Grignard reagent with a perfluoroalkyl halide is also known from Tetrahedron Letters 1996, 37(31), 5557-5560 and from CN106316777A.

Accordingly, among the presently available synthetic routes to semifluorinated alkanes two possible strategies can be distinguished. Symmetrical "triblock" semifluorinated alkanes can be prepared by dimerization of the corresponding perfluoralkyl alkylene halides. Unsymmetrical semifluorinated alkanes can only be synthesized by multistep synthesis comprising an addition reaction and further synthetic steps such as dehydration or hydrogenation.

A further problem which has not been addressed and solved in the prior art is the formation of unwanted isomeric or olefinic reaction by-products, especially in the preparation of linear semifluorinated alkanes starting from unbranched building blocks. For example,, during the synthesis of F(CF₂)₆-(CH₂)₈H, according to the methods of the prior art, a certain amount of the branched isomer F(CF₂)₆-CH(CH₃)(CH₂)₆H and F(CF₂)₆-CH=CH-(CH₂)₆H is obtained. Due to their close chemical and physical similarity it is difficult to separate such unwanted branched contaminants from crude reaction products, which is especially problematic when high chemical purities of the semifluorinated alkanes are necessary, e.g. due to regulatory requirements.

It is therefore an object of the present invention to provide a method for the synthesis of semifluorinated alkanes starting from readily available building blocks that affords the desired semifluorinated alkanes
- In high overall chemical yield and in high purity,
- with a minimum amount of unwanted, especially isomeric side products to be removed
- under reaction conditions that allow for the production of semifluorinated alkanes in technical scale
- without the use of problematic reagents
- under economically and technically favourable conditions, especially in a minimum number of consecutive steps.

Furthermore, it is an object of the present invention to provide a synthetic route to semifluorinated alkanes that offers the possibility to provide a specific product compound by reaction of alternative combinations of starting materials.

Further objects of the invention will become clear on the basis of the following description, examples, and patent claims.

### SUMMARY OF THE INVENTION

In a first aspect, the present invention provides a method for preparing a compound of formula (I)

F-(CF₂)ₙ-(CH₂)ₘ-Rₒ (I),

wherein
- n: is an integer from 2 to 12,
- m: is 2,
- Rₒ: is a linear or branched saturated alkyl and o depicts the number of carbon atoms,
- o: is an integer from 1 to 12, and wherein
- m+o: is an integer from 3 to 12;
comprising reacting a fluorinated compound of formula (II)

F-(CF₂)ₙ-(CH₂)ₘ-X (II),

wherein
- X: MgCl, MgBr, or MgI, and
- n and m: are as defined in formula (I),
with a non-fluorinated compound of formula (III)

Rₒ-Y (III),

wherein
- Y: is Cl, Br, I, and
- Rₒ: is as defined in formula (I).

Also disclosed but not part of the invention are compounds obtainable and obtained by the invention. In particular, compounds of formula (I), obtained or obtainable by a method according to the invention, as described above.

Also disclosed but not part of the invention is a composition comprising a compound of formula (I), wherein the compound of formula (I) is obtained or obtainable by a method according to the invention, preferably as a crude reaction product.

Also disclosed but not part of the invention are compounds of formula (I), obtained or obtainable by a method according to the invention, as described above, for use as a medicine.

### BRIEF DESCRIPTION OF THE DRAWINGS

Fig. 1 shows the results of further experiments conducted according to the General Reaction Procedure A as described in example 1A in summarized form.
Fig. 2 shows the results of further experiments conducted according to the General Reaction Procedure B as described in example 1B in summarized form.
Fig. 3 shows the results of further experiments conducted according to the General Reaction Procedure C as described in example 1C in summarized form.

### DETAILED DESCRIPTION OF THE INVENTION

The invention is solely defined in the claims. Some aspects of this disclosure relate to subject-matter not falling within the scope of the claims. These aspects are not part of the invention.

In the first aspect, the present invention provides a method for preparing a compound of formula (I)

F-(CF₂)ₙ-(CH₂)ₘ-Rₒ (I),

wherein
- n: is an integer from 2 to 12,
- m: is 2,
- Rₒ: is a linear or branched saturated alkyl and o depicts the number of carbon atoms,
- o: is an integer from 1 to 12, and wherein
- m+o: is an integer from 3 to 12;
comprising reacting a fluorinated compound of formula (II)

F-(CF₂)ₙ-(CH₂)ₘ-X (II),

wherein
- X: is MgCl, MgBr, or MgI, and
- n and m: are as defined in formula (I),
with a non-fluorinated compound of formula (III)

Rₒ-Y (III),

wherein
- Y: is Cl, Br, I, ,and
- Rₒ: is as defined in formula (I).

The method according to this first aspect of the invention relates to the synthesis of semifluorinated alkanes of the general formula F-(CF₂)ₙ-(CH₂)ₘ-Rₒ (I), wherein index n is an integer from 2 to 12 and index m is 2. The substituent Rₒ in formula (I) is a linear or branched saturated alkyl group and o depicts the number of carbon atoms and o is an integer from 1 to 12. Accordingly, the substituent Rₒ may be a linear or branched saturated alkyl group with 1 to 12 carbon atoms such as, for example, methyl, ethyl, n-propyl, iso-propyl, n-butyl, sec-butyl, isobutyl, tert-butyl, n-pentyl, isopentyl, neopentyl, n-hexyl, n-heptyl, n-nonyl, n-decyl, n-undecyl and n-dodecyl.

In a preferred embodiment, the group Rₒ is a linear saturated alkyl group with 1 to 12 carbon atoms, namely methyl, ethyl, n-propyl, n-butyl, n-pentyl, n-hexyl, n-heptyl, n- octyl, n-nonyl, n-decyl, n-undecyl and n-dodecyl. In further preferred embodiments, the group Rₒ is a linear or branched saturated alkyl group with 3 to 12 carbon atoms corresponding to the index o being an integer from 3 to 12. In further preferred embodiments Rₒ is a linear saturated alkyl group with 3 to 12 carbon atoms, namely n-propyl, n-butyl, n-pentyl, n-hexyl, n-heptyl, n-octyl, n-nonyl, n-decyl, n-undecyl or n-dodecyl.

In further embodiments, index o is an integer from 1 to 9, more preferably 1 to 8. In a preferred embodiment the group Rₒ is a linear saturated alkyl group with 1 to 9, preferably 1 to 8, carbon atoms. In some embodiments Rₒ is a linear or branched alkyl group with 1 to 5 carbon atoms.

In further embodiments, index o is an integer in the range from 3 to 8, more preferably in the range of 3 to 5, corresponding to a saturated alkyl group with 3 to 8 or preferably 3 to 5 carbon atoms as described above. Index m depicts the number of methylene or CH₂-groups directly attached to the fluorinated part of the products of general formula (I). Index m is 2.

Furthermore, in the compounds of formula (I) according to the present invention the sum of indexes m and o (corresponding to m+o) is an integer from 3 to 12, namely 3, 4, 5, 6, 7, 8, 9, 10, 11 or 12. The sum of indexes m and o (m+o) in general formula (I) depicts the total number of carbon atoms in the non-fluorinated alkyl part of the products of formula (I) according to the present invention. In preferred embodiments m+o is an integer from 4 to 10. In some preferred embodiments m+o is an integer from 3 to 9. In further preferred embodiments the sum of m and o is 5 to 8. In even more preferred embodiments, m+o is 5, 6 or 8.

The index n in the products of formula (I) of the method according to the present invention may be an integer from 2 to 12, namely 2, 3, 4, 5, 6, 7, 8, 9, 10, 11 or 12. Index n depicts the number of carbon atoms in the fluorinated part of the products of general formula (I). In some embodiments, the index n is an integer from 2 to 7. In preferred embodiments, index n is an integer from 4 to 8, in even more preferred embodiments index n in the products according to general formula (I) is one from 4 to 6. Most preferably, n is 4 or 6.

An alternative nomenclature for some semifluorinated alkanes according to formula (I) of the present invention, especially for the linear semifluorinated alkanes, is based on the general formula FxHy, wherein F means the linear perfluorinated hydrocarbon segment, H means the linear non-fluorinated hydrocarbon segment and x, y is the number of carbon atoms of the respective segment. For example, F4H5 may be used to denote 1-perfluorobutyl-pentane or CF₃(CF₂)₃-(CH₂)₄CH₃ (which may be also, alternatively expressed as formula F(CF₂)₄(CH₂)₅H), which has a linear perfluorinated segment F with four carbons (x = 4) and a linear non-fluorinated hydrocarbon segment with five carbons (y = 5). Furthermore, F6H8 may be used to denote 1-perfluorohexyl-octane or CF₃(CF₂)₅-(CH₂)₇CH₃ (which may be also, alternatively expressed as formula F(CF₂)₆(CH₂)₈H), which has a linear perfluorinated segment F with six carbons (x = 6) and a linear non-fluorinated hydrocarbon segment with 8 carbons (y = 8).

Accordingly, the semifluorinated alkanes according to formula (I) of the present invention may comprise, but are not limited to, for example: F(CF₂)₄-(CH₂)₅H (F4H5), F(CF₂)₄-(CH₂)₆H (F4H6), F(CF₂)₄-(CH₂)₇H (F4H7), F(CF₂)₄-(CH₂)₈H (F4H8), F(CF₂)₅-(CH₂)₅H (F5H5), F(CF₂)₅-(CH₂)₆H (F5H6), F(CF₂)₅-(CH₂)₇H (F5H7), F(CF₂)₅-(CH₂)₈H (F5H8), F(CF₂)₆-(CH₂)₅H (F6H5), F(CF₂)₆-(CH₂)₆H (F6H6), F(CF₂)₆-(CH₂)₇H (F6H7), F(CF₂)₆-(CH₂)₈H (F6H8) and F(CF₂)₈-(CH₂)₈H (F8H8). More preferably, said semifluorinated alkane according to formula (I) may be selected from F(CF₂)₄-(CH₂)₅H (F4H5), F(CF₂)₄-(CH₂)₆H (F4H6), F(CF₂)₆-(CH₂)₆H (F6H6), F(CF₂)₅-(CH₂)₈H (F6H8) and F(CF₂)₈-(CH₂)₈H (F8H8).

As outlined above, the method according to this aspect of the invention comprises reacting a fluorinated compound of formula (II)

F-(CF₂)ₙ-(CH₂)ₘ-X (II),

with a non-fluorinated compound of formula (III)

Rₒ-Y (III),

wherein n and m are as defined in formula (I), and wherein Rₒ is as defined in formula (I). In the starting material according to formula (II) the substituent X is a magnesium halide selected from MgCl, MgBr or MgI. The starting material is an organometallic, or more specifically, organomagnesium compound with the substituent MgCl, MgBr or MgI attached as the substituent X. Such compounds are known to those of skill in the art as Grignard compounds or reagents and may be prepared by standard techniques starting from the corresponding halogenated precursors (the compounds of formula (I) with X being either Cl, Br or I) and, for example, reaction with metallic magnesium or by transmetallation with another readily available Grignard reagent such as ethyl magnesium chloride or iso-propyl magnesium chloride.

The compounds of formula (II) as described above are reacted with a non-fluorinated compound of general formula (III)

Rₒ-Y (III),

wherein the group Rₒ has the same meaning as defined above for the products according to general formula (I) with the index o depicting the number of carbon atoms in this non-fluorinated starting material. In this non-fluorinated starting material according to formula (III) the substituent Y is a halogen selected from Cl, Br or I The non-fluorinated starting material according to formula (III) is be a halogenide, having a halogen atom other than fluorine attached to one end of the molecule.

This means that only one of the two starting materials of general formulas (II) or (III), respectively, may be an organomagnesium compound having the substituent MgCl, MgBr or MgI, whereas the other starting material has to be the halogenated compound having the substituent Cl, Br or I.

In the invention, the starting material of formula (II) comprises the organomagnesium halide. Preferably, if the substituent Y of formula (III) is Cl, Br, or I, then in the other starting material of formula (II) the substituent X is selected from the group consisting of MgCl, MgBr and MgI. In an even more preferred embodiment of the invention the substituent X of the starting material according to formula (II) is MgCl.

In the preferred embodiments, wherein the substituent X in the starting material of formula (II) is MgCl, MgBr or MgI, in the other starting material of formula (III) the substituent Y is selected from the group consisting of Cl, Br and I. In an even more preferred embodiment of the Invention, the substituent Y in formula (III) is I.

Any combination of X in formula (II) and Y in formula (III) is suitable, as long as either X in formula (II) or Y in formula (III) is a magnesium halide and the other is a halogenide. Only combinations wherein X in formula (II) is a magnesium halide are part of the invention. The following table lists all combinations.

Table 1: Combinations of X in starting materials of formula (II) and Y in starting materials of formula (III). Only combinations wherein X in formula (II) is a magnesium halide are part of the invention.

| **formula (II)** | **formula (III)** |
|---|---|
| X = MgCl | Y = Cl |
| X = MgCl | Y = Br |
| X = MgCl | Y=I |
| X = MgBr | Y = Cl |
| X = MgBr | Y = Br |
| X = MgBr | Y = I |
| X = MgI | Y = Cl |
| X = MgI | Y = Br |
| X = MgI | Y = I |

The starting materials of formula (II) and formula (III) may be used either in equimolar amounts or in a broad range of amounts relative to each other, in which either one of the two selected starting materials may be used in a molar excess relative to the other. In preferred embodiments of the present invention, however, the organomagnesium compound of either formula (II) is used in molar excess relative to halide of formula (III), respectively. In further preferred embodiments of the invention, the fluorinated compound of formula (II), wherein X is MgCl, MgBr or MgI, is used in an amount of 1.0 to 10.0, preferably of 1.0 to 4.0, and more preferably of 1 to 2 mol-equivalents with regard to amount of compound of formula (III), wherein Y is Cl, Br or I.

In even more preferred embodiments of the invention, the organomagnesium halide compound of either formula (II) is used in an amount of 1.0, 1.3, 1.5, 1.6 or 2 mol-equivalents compared to the other compound. Accordingly, in a preferred embodiment of the invention, the compound of formula (II), wherein X is MgCl, MgBr or MgI, is used in an amount of 1, 1.3, 1.5, 1.6 or 2 mol-equivalents with regard to amount of compound of formula (III), wherein Y is Cl, Br or I.

In a preferred embodiment of the present invention, X is MgCl and Y is iodine, respectively in the compounds of formula (II) and (III). In a more preferred embodiment of the present invention, X is MgCl and Y is iodine. In a most preferred embodiment of the present invention, in the compounds of formula (II) and (III), X is MgCl and Y is iodine, respectively, and the compound of formula (II) is used in an amount of 1 to 2 mol-equivalents with regard to the amount of compound of formula (III).

The method as described above suitable for the synthesis of a variety of compounds, which all within the definition of formula (I) as outlined in detail above:

F-(CF₂)ₙ-(CH₂)ₘ-Rₒ (I),

wherein
- n: is an integer from 2 to 12,
- m: is 2,
- Rₒ: is a linear or branched saturated alkyl and o depicts the number of carbon atoms,
- o: is an integer from 1 to 12, and wherein
- m+o: is an integer from 3 to 12.

Independently, of whether the indices refer to formula (I), formula (II) or formula (III) the following values and combinations for m, n and o are particularly preferred:
In some preferred embodiments of the present invention, index n depicting the number of carbon atoms in the fluorinated part of the starting material of formula (II) or of the products of formula (I) is an integer from 2 to 8. In some embodiments n is 2 to 7. In a more preferred embodiment n is an integer from 4 to 6. In a specific embodiment n is 4, 5 or 6. In particular preferred embodiments n is 4 or 6.

In the present invention, m is 2.

In some preferred embodiments of the invention, the index o depicting the number of carbon atoms in the linear or branched, preferably linear saturated rest Rₒ according to formula (I) or (III) is an integer from 2 to 10. In some embodiments o is an integer from 1 to 9. In a more preferred embodiment of the invention, o is an integer from 3 to 8. In one more preferable embodiment of the invention, index o is an integer of 3 to 5. In an alternative preferred embodiment of the invention, index o is an integer of 6 to 8. In one most preferred embodiment o is 3. In an alternative most preferred embodiment, o is 6.

In a preferred embodiment Rₒ is a linear saturated alkyl group.

As outlined above, the sum of indexes m and o (m+o) in general formula (I) depicts the total number of carbon atoms in the non-fluorinated alkyl part of the products of formula (I) according to the present invention. In some preferred embodiments m + o is an integer from 3 to 9. In preferred embodiments, m+o is an integer of 3 to 10. In a more preferred embodiment, m+o is an integer of 4 to 8. In an even more preferred embodiment of the invention m+o is an integer of 5 to 8. In a particularly preferred embodiment of the invention, the sum of indexes m+o is 5. In a further particularly preferred embodiment, the sum of indexes m+o is 8.

Further preferred embodiments of the present invention relate to preferred combinations of the indexes n, m and o as well as the sum of m+o as described above. In one preferred embodiment, index n is an integer from 4 to 6, m is 2, o is an integer from 1 to 8 and m+o is an integer of 4 to 8. In a specific preferred embodiment n is 4, m is an integer from 0 to 4, o is an integer from 1 to 5 and m + o is 5. In an even more preferred specific embodiment of the present invention, n is 4, m is 2 and o is 3.

In a further specific preferred embodiment of the invention, n is 6, m is 2, o is an integer of 1 to 8 and m+o is 8. In a most preferred specific embodiment of the present invention, n is 6, m is 2 and o is 6.

The method of the invention may be carried out in the presence of a transition metal compound. The transition metal compound according to the present invention may be a catalyst to catalyze the reaction of the starting material of formula (II) with the other starting material of formula (III) to form the semifluorinated alkane of formula (I).

Therefore, in one embodiment of the invention, the reaction is carried out in presence of a transition metal compound, wherein said transition metal compound is present in a catalytic amount.

Suitable transition metal catalysts are known to those of skill in the art. In the present invention, suitable transition metal compounds are preferably compounds comprising a transition metal atom of group 8, 9, 10 or 11, preferably of group 9, 10 or 11, more preferably of group 11 of the periodic table of the elements. Preferably, the transition metal compound comprises the transition metal in ionic or salt form.

In further preferred embodiments of the invention, the transition metal compound comprises at least one, preferably one transition metal atom which is selected from the group consisting of Cu, Ni, Co, Pd and Fe. In even more preferred embodiments, the transition metal compound is a copper (Cu) compound, preferably a Cu(I) or Cu(II) comprising compound.

The transition metal compound according to the present invention may preferably be a transition metal halide or a transition metal triflate or a transition metal acetylacetonate. In a preferred embodiment of the invention, the transition metal compound is selected from the group consisting of CuCl₂, CuBr₂, Cu(OTf)₂, CuI, NiCl₂, Ni(acac)₂, CoCl₂, PdCl₂. FeCl₃, AgNO₃, AuCl₃, CuBr, Cu(OTf), Rh(acac)(C₂H₄)₂, [RhCl(C₂H₄)₂] and RhCl₃, preferably selected from the group consisting of CuCl₂, CuBr₂, Cu(OTf)₂, CuI, NiCl₂, Ni(acac)₂, CoCl₂, PdCl₂ and FeCl (wherein "OTf" denotes a triflate and "acac" denotes an acetylacetonate). In a most preferred embodiment of the present Invention, the transition metal compound is CuCl₂.

The transition metal compound may be used either in stochiometric or equimolar amounts to either one of the starting materials of formulas (II) or (III) or preferably in less than stochiometric amounts relative to the starting materials.

In general, however, a catalytic amount is a substoichiometric amount. Accordingly, the transition metal compound is preferably present in substoichiometric amounts compared to the compounds of either formula (II) and/or (III) .

In a particular embodiment of the invention, the transition metal compound is present in an amount of 0.01 to 0.2, preferably in an amount of 0.02 to 0.1, more preferably in an amount of 0.03 to 0.05 mol-equivalents with regard to the amount of compound of formula (III), wherein X in formula (II) is MgCl, MgBr or MgI and Y in formula (III) is Cl, Br or I.

In a further preferred embodiment, the method of the invention is carried out in the presence of a 1,3-diene compound. Preferably, said 1,3-diene compound is present in the reaction in addition to the transition metal compound. Accordingly, in a preferred embodiment, the present invention refers to a method for preparing a compound of formula (I) a described above, comprising reacting a fluorinated compound of formula (II) as described above with a non-fluorinated compound of formula (III) as described above in the presence of a transition metal compound and in the presence of a 1,3-diene compound.

The term "1,3-diene compound" as used in here is to be understood broadly and may be any organic compound with two conjugated olefinic double bonds that is suitable to be used in the method of the present invention. Suitable 1,3-diene compounds are known to the person skilled in the art. Preferred non-limiting examples for 1,3-diene compounds include 1,3-butadiene, isoprene, 1,3-pentadiene, 1,3-cyclohexadiene, 1,5-cyclooctatetraene, cis/trans-stilbene, styrol, 2,3-dimethylbutadiene, benzonitrile, phenylacetylene and derivatives thereof.

In a preferred embodiment of the invention the 1,3-diene compound is selected from the group consisting of 1,3-butadiene, isoprene, 1,3-pentadiene, 1,3-cyclohexadiene, 1,5-cyclooctatetraene, cis/trans-stilbene, styrol, 2,3-dimethylbutadiene, benzonitrile, phenylacetylene and derivatives thereof. In a more preferred embodiment of the invention the 1,3-diene compound is selected from the group consisting of 1,3-butadiene, isoprene, 1,3-pentadiene, 1,3-cyclohexadiene, 1,5-cyclooctatetraene and 2,3-dimethylbutadiene.

In an alternative embodiment, instead of an 1,3-diene compound, a nonconjugated diene compound such as, for example, 1,4-cyclohexadiene can be used.

In an even more preferred embodiment of the invention, the 1,3-diene compound is 1,3-butadiene or isoprene. In a most preferred embodiment of the invention the 1,3-diene compound is isoprene.

The 1,3-diene compound might be present in any suitable amount. In some embodiments of the invention, the 1,3-diene compound is used in equimolar amounts, in excess or in substochiometric amounts with regard the compound of formula (II) or (III).

In a preferred embodiment of the invention the 1,3-diene compound is present in an equimolar amount or in excess with regard to the halogenide compound.

In a specific preferred embodiment of the invention, wherein in the starting material of formula (II) the substituent X is MgCl, MgBr or MgI and Y in the starting material of formula (III) is Cl, Br or I, the 1,3-diene compound is used in an amount of 0.5 to 10 molar equivalents, preferably in an amount of 1.0. to 5.0 molar equivalents and even more preferably in an amount of 1.0 to 2.0 molar equivalents with regard to the amount of compound of formula (III).

In more preferred embodiments of the invention, the reaction is carried out in the presence of a transition metal compound and a 1,3-diene compound. In some embodiments of the invention the transition metal compound is a copper (Cu) compound, preferably a Cu(I) or Cu(II) comprising compound and the 1,3-diene compound is selected from the group comprising 1,3-butadiene, isoprene, 1,3-pentadiene, 1,3-cyclohexadiene, 1,5-cyclooctatetraene, cis/trans-stilbene, styrol, 2,3-dimethylbutadiene, benzonitrile, phenylacetylene and derivatives thereof.

In a more preferred embodiment of the invention, the reaction is carried out in the presence of a transition metal compound selected from the group consisting of CuCl₂, CuBr₂, Cu(OTf)₂, CuI, NiCl₂, Ni(acac)₂, CoCl₂, PdCl₂. FeCl₃, AgNO₃, AuCl₃, CuBr, Cu(OTf), Rh(acac)(C₂H₄)₂, [RhCl(C₂H₄)₂] and RhCl₃, preferably selected from the group consisting of CuCl₂, CuBr₂, Cu(OTf)₂, CuI, NiCl₂, Ni(acac)₂, CoCl₂, PdCl₂ and FeCl and the 1,3-compound is selected from the group consisting of 1,3-butadiene, isoprene, 1,3-pentadiene, 1,3-cyclohexadiene, 1,5-cyclooctatetraene, cis/trans-stilbene, styrol, 2,3-dimethylbutadiene, benzonitrile, phenylacetylene and derivatives thereof.

In an even more preferred embodiment of the invention, the reaction is carried out in the presence of a transition metal compound selected from the group consisting of CuCl₂, CuBr₂ Cu(OTf)₂, Ni(acac)₂, NiCl₂, CoCl₂, PdCl₂. FeCl₃ and the 1,3-diene compound is selected from the group consisting of 1,3-butadiene or isoprene. In a most preferred embodiment of the invention, the transition metal compound is CuCl₂ and the 1,3-diene compound is isoprene.

In some embodiments of the invention, the fluorinated starting material of formula (II) is reacted with the non-fluorinated compound of formula (III) in the presence of an alkali halide or alkaline earth metal halide. Preferably, the compound of formula (II) is reacted with the compound of formula (III) in the presence of a lithium or magnesium halide such as, for example MgCl₂. In a more preferred embodiment of the invention, the compound of formula (II) is reacted with the compound of formula (III) in the presence of a lithium halide selected from the group consisting of LiCl, LiBr and LiI. In a most preferred embodiment of the invention the lithium halide is lithium iodide (LiI).

The alkali or alkaline earth metal halide, preferably the alkali metal halide, might be used in any suitable concentration. Preferably, the alkaline or alkaline earth metal halide is a lithium or magnesium halide, wherein the lithium or magnesium halide is used in an amount of up to 0.1 molar equivalents, preferably in an amount of up to 0.04 molar equivalents with regard to the amount of compound of formula (II).

It is clear to the person skilled in the art, that the method of the invention is suitable for the synthesis of several different compounds of formula (I). The method of the invention is particularly suitable for the synthesis of the following preferred compounds:

F-(CF₂)₄-(CH₂)₄-CH₃ (F4H5) (compound (Ia))

F-(CF₂)₄-(CH₂)₅-CH₃ (F4H6)

F-(CF₂)₆-(CH₂)₅-CH₃ (F6H6)

F-(CF₂)6-(CH₂)₇-CH₃ (F6H8) (compound (Ib))

These compounds might be synthesized using the method of the invention, with different compounds of formula (II) and (III). Accordingly, in one embodiment, the invention relates to a method for preparing a compound of formula (I), wherein the product compound according to formula (I) is selected from the group consisting of F-(CF₂)₄-(CH₂)₄-CH₃ (compound (Ia), F4H5), F-(CF₂)₄-(CH₂)₅-CH₃ (F4H6), F-(CF₂)₆-(CH₂)₅-CH₃ (F6H6) and F-(CF₂)6-(CH₂)₇-CH₃ (compound (Ib), F6H8).

In a preferred embodiment, the invention relates to a method for preparing a compound of formula (I), wherein the compound of formula (I) is F(CF₂)₄-(CH₂)₄-CH₃ (compound (Ia)), F4H5) prepared by reacting CF₃(CF₂)₃-(CH₂)ₘ-X as the compound of formula (II) with Rₒ-Y as the compound of formula (III), wherein
- m: is 2
- o: is an integer of 1 to 5, and
- m+o: is 5; and wherein
- X: is MgCl, MgBr, or MgI, and
- Y: is Cl, Br, I,

In a preferred embodiment, the compound of formula (I) is F(CF₂)₄-(CH₂)₄-CH₃ (compound (Ia), F4H5) and is prepared by reacting F(CF₂)₄-(CH₂)₂-Mg-Cl, F(CF₂)₄-(CH₂)₂-Mg-Br or F(CF₂)₄-MgI as the compound of formula (II) with Cl-(CH₂)₂-CH₃, Br-(CH₂)₂-CH₃ or I-(CH₂)₂-CH₃ as the compound of formula (III), optionally in the presence of a 1,3-diene compound and/or a transition metal compound.

In a specific preferred embodiment of the invention, the compound of formula (I) is F(CF₂)₄-(CH₂)₄-CH₃ (compound (Ia), F4H5) and is prepared by reacting F(CF₂)₄-(CH₂)₂-Mg-Cl as the compound of formula (II) with I-(CH₂)₂-CH₃ as the compound of formula (III).

In a more preferred specific embodiment of the invention, the compound of formula (I) is F(CF₂)₄-(CH₂)₄-CH₃ (compound (Ia), F4H5) and is prepared by reacting F(CF₂)₄-(CH₂)₂-Mg-Cl as the compound of formula (II) with I-(CH₂)₂-CH₃ as the compound of formula (III) in the presence of a 1,3-diene compound and/or a transition metal compound.

In a more preferred embodiment of the invention. the compound of formula (I) is F(CF₂)₄-(CH₂)₄-CH₃ (compound (Ia), F4H5) and is prepared by reacting F(CF₂)₄-(CH₂)₂-Mg-Cl as the compound of formula (II) with I-(CH₂)₂-CH₃ as the compound of formula (III) in the presence of isoprene or 1,3-butadiene and/or a copper compound, preferably CuCl₂.

In an even more preferred embodiment of the invention. the compound of formula (I) is F(CF₂)₄-(CH₂)₄-CH₃ (compound (Ia), F4H5) and is prepared by reacting F(CF₂)₄-(CH₂)₂-Mg-Cl as the compound of formula (II) with I-(CH₂)₂-CH₃ as the compound of formula (III) in the presence of isoprene and CuCl₂. In a further more preferred embodiment of the invention. the compound of formula (I) is F(CF₂)₄-(CH₂)₄-CH₃ (compound (Ia), F4H5) and is prepared by reacting F(CF₂)₄-(CH₂)₂-Mg-Cl as the compound of formula (II) with I-(CH₂)₂-CH₃ as the compound of formula (III) in the presence of 1,3-butadiene and CuCl₂.

In a further preferred embodiment, the invention relates to a method for preparing a compound of formula (I), wherein the compound of formula (I) is F(CF₂)₆-(CH₂)₇-CH₃ (compound (Ib), F6H8) prepared by reacting CF₃(CF₂)₆-(CH₂)ₘ-X as the compound of formula (II) with Rₒ-Y as the compound of formula (III), wherein
- m: is 2,
- o: is an integer from 1 to 8, and wherein
- m+o: is 8
- X: is MgCl, MgBr, or MgI, and
- Y: is Cl, Br, I,

In an additional particularly preferred embodiment of the invention, the compound of formula (I) is F(CF₂)₆-(CH₂)₇-CH₃ (compound (Ib), F6H8) and is prepared by reacting F(CF₂)₆-(CH₂)₂-Mg-Cl, F(CF₂)₆-(CH₂)₂-Mg-Br or F(CF₂)₆-(CH₂)₂-Mg-I as the compound of formula (II) with Cl-(CH₂)₅-CH₃, Br-(CH₂)₅-CH₃ or I-(CH₂)₅-CH₃ as the compound of formula (III), optionally in the presence of a 1,3-diene compound and/or a transition metal compound.

In a preferred specific embodiment of the invention, the compound of formula (I) is F(CF₂)₆-(CH₂)₇-CH₃ (compound (Ib), F6H8) and is prepared by reacting F(CF₂)₆-(CH₂)₂-Mg-Cl as the compound of formula (II) with I-(CH₂)₅-CH₃ as the compound of formula (III).

In a more preferred embodiment of the invention, the compound of formula (I) is F(CF₂)₆-(CH₂)₇-CH₃ (compound (Ib), F6H8) and is prepared by reacting F(CF₂)₆-(CH₂)₂-Mg-Cl as the compound of formula (II) with I-(CH₂)₅-CH₃ as the compound of formula (III) in the presence of a 1,3-diene compound and/or a transition metal compound.

In a further preferred embodiment, the compound of formula (I) is F(CF₂)₆-(CH₂)₇-CH₃ (compound (Ib), F6H8) and is prepared by reacting F(CF₂)₆-(CH₂)₂-Mg-Cl as the compound of formula (II) with I-(CH₂)₅-CH₃ as the compound of formula (III) in the presence of isoprene or 1,3-butadiene and/or a copper compound, preferably CuCl₂.

In a yet more preferred embodiment of the invention, the compound of formula (I) is F(CF₂)₆-(CH₂)₇-CH₃ (compound (Ib), F6H8) and is prepared by reacting F(CF₂)₆-(CH₂)₂-Mg-Cl as the compound of formula (II) with I-(CH₂)₅-CH₃ as the compound of formula (III) in the presence of isoprene and CuCl₂.An alternative preferred embodiment of the invention, the compound of formula (I) is F(CF₂)₆-(CH₂)₇-CH₃ (compound (Ib), F6H8) and is prepared by reacting F(CF₂)₆-(CH₂)₂-Mg-Cl as the compound of formula (II) with I-(CH₂)₅-CH₃ as the compound of formula (III) in the presence of 1,3-butadiene and CuCl₂.

The method of the invention might be performed in any suitable reaction vessel. Preferably, a suitable reaction vessel allows to hold the liquid or at least partly liquid or dissolved starting materials and reagents present in the reaction and may be used, for example, in form of a vessel, bottle flask or reactor which allows the starting materials and reagents to be brought in contact with each other and, preferably, to control the temperature of the reaction mixture. The reaction according to the present invention may be performed in a single reaction vessel or in a plurality of reaction vessel in parallel or consecutive manner. Preferably however, the reaction is performed in a single reaction vessel, preferably in a single reaction vessel, into which all starting materials, reagents and/or solvents present in the reaction are added (herein also referred to as a "one pot synthesis").

Accordingly, in one step, often in the first step, the method according to the present invention comprises the step of:
- providing a reaction vessel.

In a further step, often in the second step, the method according to the present invention for producing semifluorinated alkanes of the general formula (I) as described above comprises the step of:
- adding the compound of formula (III), wherein Y is Cl, Br or I, to the reaction vessel.

In this step of the method according to the present invention the chosen starting materials of formula (III) having a halogen atom selected from the group consisting of Cl, Br and I as the substituent Y are added to the reaction vessel. In a further embodiment, the method of the present invention comprises the step of:
- adding the compound of formula (II), wherein X is MgCl, MgBr or MgI to the reaction vessel.

According to this step of the present invention, the selected starting material of compounds (II) having a magnesium halide selected from the group consisting of MgCl, MgBr and MgI are added to the reaction vessel. The selected magnesium halide may be added to the reaction vessel as the first component of the reaction mixture or, in other words may be added before the corresponding chosen halogenated compound of formula (III) wherein Y is either Cl, Br or I. Alternatively, however, it is also possible to add the chosen halogenated compound of formula (III) wherein Y is either Cl, Br or I in the first step and to consecutively add the chosen compound of formula (II) having a magnesium halide selected from the group consisting of MgCl, MgBr and MgI in a further step.

In other embodiments of the method according to the present invention, the compound of formula (II) and the compound of formula (III) may be added at least partly simultaneously. According to this embodiment, the chosen starting materials of formulas (II) and (III), one being the magnesium halide and one being the halogenated starting material are not added to reaction vessel in an entirely subsequent order. Accordingly, it is possible to add one of the two starting materials first, and then to start adding the other starting material before the addition of the first starting material has been completed. In this embodiment also, the order of addition of the reagents of formulas (II) or (III) is not critical and may be selected according to the specific compounds to react with each other.

Furthermore, the compounds of formulas (II) and (III), as well as all other liquid compounds or reagents to be added to the reaction vessel or the reaction mixture ay be added at any rate suitable to realize controlled reaction conditions, especially controlled temperature of the reaction mixture. However, the reagents and starting materials may be added at once or over a prolonged period of time. Preferably, the liquid reagents and starting materials or solutions thereof are added dropwise to the reaction vessel or reaction mixture.

In a further embodiment of the present invention the compound of formula (II), wherein X is MgCl, MgBr, or MgI, is prepared in situ. The term "in situ" as used herein means that the magnesium halide of formula (II) is formed in an additional step prior to the reaction with or addition of the corresponding second starting material which is the starting material with X selected from Cl, Br and I. Preferably the in situ formation of the magnesium halogenide of formula (II) with X being MgCl, MgBr or MgI is performed in the same reaction vessel in which the reaction with the corresponding other starting material of formula (III) in which Y is Cl, Br or I is conducted.

According to this embodiment, the compound of formula (II), wherein X is MgCl, MgBr or MgI are added to the reaction vessel in form of their halogenated precursors followed by treatment with a further Grignard reagent. The term "a further Grignard reagent" as used herein means a Grignard reagent which is used to prepare the magnesium halide of formula (II) or formula (III) to be used as one of the starting materials in the method of the present invention by transmetallation or, in other words, transfer of the magnesium halide substituent of the further Grignard reagent to the halogenated precursor of the chosen starting material of formula (II) or (III) .

As an example, a starting material of formula (II) in which X is MgCl can be prepared by treatment of the corresponding halogenated starting material of the same formula (II) in which X is Cl, Br, or I, preferably Br or I, and more preferably I, with a further Grignard reagent having a MgCl-substituent. In the same way, as a further example, a starting material of formula (II) in which X is MgBr can be prepared by treatment of the corresponding halogenated starting material of the same formula (II) in which X or Y is Cl, Br, or I, preferably Br or I, and more preferably I, with a further Grignard reagent having a MgBr-substituent.

The further Grignard reagent as used in this in situ preparation of the magnesium halide starting material of either formula (II), preferably is chosen from readily available Grignard reagents which, preferably, are commercially available in form of solutions in suitable solvents such as hexanes or THF. Examples of readily available further Grignard reagents comprise but are not limited to isopropylmagnesium chloride, isopropylmagnesium bromide, ethylmagnesium chloride, ethylmagnesium bromide, phenylmagnesium chloride and phenylmagnesium bromide, preferably isopropylmagnesium chloride, isopropylmagnesium bromide, ethylmagnesium chloride, ethylmagnesium bromide.

Accordingly, in a further embodiment of the method of the present invention the compound of formula (II), wherein X is MgCl, MgBr or MgI, is added in form of their halogenated precursors followed by treatment with a further Grignard reagent selected from the group consisting of isopropylmagnesium chloride, isopropylmagnesium bromide, ethylmagnesium chloride, ethylmagnesium bromide, phenylmagnesium chloride, phenylmagnesium bromide, preferably selected from the group consisting of isopropylmagnesium chloride, isopropylmagnesium bromide, ethylmagnesium chloride, ethylmagnesium bromide, thereby generating the compound of formula (II), wherein X is MgCl, MgBr or MgI, in situ.

In a further embodiment, the method of the present invention comprises as further steps:
- adding the transition metal compound to the reaction vessel, and/or
- adding the 1,3-diene compound to the reaction vessel.

In this embodiment also, both steps can be performed independently of each other and in any suitable order. For example, the transition metal compound can be added to the reaction vessel or the reaction mixture first, followed by addition of the chosen 1,3-diene compound as described above, or vice versa. As an alternative, both reagents can be added simultaneously, either before, during or after the addition of the starting materials of formulas (II) or (III) or further reagents.

In yet a further embodiment, the present method for the preparation of semifluorinated alkanes of formula (I) optionally comprises the step of
- adding a lithium or magnesium halide to the reaction vessel.

The lithium or magnesium halide as described above, preferably the lithium halide and more preferably LiI (lithium iodide) may also be added to the reaction vessel or reaction mixture at any suitable time either together with or before or after the other chosen starting material and reagents.

In preferred embodiments, the method according to the present invention comprises the steps of
- adding the compound of formula (III), wherein Y is Cl, Br or I, to the reaction vessel, and
- adding the transition metal compound and the 1,3-diene compound to the reaction vessel, and
- optionally adding a lithium or magnesium halide to the reaction vessel; and subsequently
- adding respectively the compound of formula (II), wherein X is MgCl, MgBr or MgI, to the reaction vessel.

In other preferred embodiments, the method according to the present invention comprises the steps of
- adding the compound of formula (II), wherein X is MgCl, MgBr or MgI, and
- adding the transition metal compound and the 1,3-diene compound to the reaction vessel, and
- optionally adding a lithium or magnesium halide to the reaction vessel; and subsequently
- adding respectively the non-fluorinated compound of formula (III), wherein Y is Cl, Br or I, to the reaction vessel.

In other specific preferred embodiments, the method according to the present invention comprises the steps of
- adding the compound of formula (II), wherein X is MgCl, MgBr or MgI to the reaction vessel, and
- adding the transition metal compound and the 1,3-diene compound to the reaction vessel, and
- optionally adding a lithium or magnesium halide to the reaction vessel; and subsequently
- adding the fluorinated compound of formula (III), wherein Y is Cl, Br or I, to the reaction vessel.

In further specific preferred embodiments, the method according to the present invention comprises the steps of
- adding the fluorinated compound of formula (III), wherein Y is Cl, Br or I, to the reaction vessel, and
- adding the transition metal compound and the 1,3-diene compound to the reaction vessel, and
- optionally adding a lithium or magnesium halide to the reaction vessel; and subsequently
- adding the compound of formula (II), wherein X is MgCl, MgBr or MgI, to the reaction vessel.

In yet further embodiments, the method for the preparation of semifluorinated alkanes of formula (I) as described above further comprises the step of
- isolating the compound of formula (I) in form of a crude reaction product (mixture).

According to this embodiment, the semifluorinated alkanes of formula (I) are usually formed as the main reaction product of the reaction of a compound of formula (II) with a compound of formula (III), often as the dominating reaction product among minor amounts of side-products, as described further below, as well as among further reagents present in the reaction mixture. Usually, after termination of the reaction of the compounds of formulas (II) and (III), either after complete turnover or after the reaction has been terminated actively, for example, by addition of a protic solvent and/or water, the reaction mixture is usually subjected to standard workup procedures, such as extractive workup procedures, during which all or the majority of inorganic side-products can be removed. Accordingly, in some embodiments, the crude reaction product is isolated after extractive workup and removal of solvents and low-boiling by-products.

During the reaction of the chosen compounds of formulas (II) and (III) to form the semifluorinated alkanes of formula (I), optionally in the presence of a transition metal compound and optionally in the presence of a 1,3-diene compound, the temperature of the reaction mixture may be controlled over a broad temperature range, preferably at temperatures in the range of from about -72°C to about 70°C, preferably in the range of from about -20°C to about 25°C and more preferably at temperatures around about 0°C, depending on the kind and reactivity of the specific starting materials and reagents chosen.

In preferred embodiments of the method of the present invention, however, the addition of the compounds of formula (II) and/or (III), and/or optionally the addition of the transition metal compound and the 1,3-diene compound and/or optionally the addition of a lithium or magnesium halide is carried out with cooling of the reaction mixture, preferably at a temperature in the range of about -72°C to about 0°C, more preferably in the range of -20°C to about 0°C, more preferably at a temperature of about 0°C. It should be understood that the temperature or temperature range chosen during the addition of the above-mentioned reagents has to be kept constant during the reaction. In preferred embodiments, the reagents as mentioned above are added at a certain temperature, usually in the range of from about -72°C to about 0°C and is then allowed to raise gradually to temperatures in the range of about 0° to about 70°C, often to about 23°C, 50°C or 70°C, if necessary under heating of the reaction mixture.

The method according to the present invention may be preferably performed in the presence of a solvent or a mixture of two or more different solvents. Preferably, the solvent or solvents may be chosen from aprotic organic solvents which are compatible to organometallic compound such as the magnesium halides of formulas (II) with X being MgCl, MgBr or MgI or the further Grignard-reagents as described above. Accordingly, in preferred embodiments of the method of the present invention, the reaction of the compound of formula (II) with the compound of formula (III), optionally in the presence of a 1,3-diene compound and in the presence of a transition metal compound, is conducted in the presence of a solvent selected from the group consisting of THF, dioxane, diethylether, monoglyme, diglyme, pentane, n-hexane, heptane, petrolether, 2-methyltetrahydrofuran and hexanes, preferably the reaction is performed in the presence of n-hexane or hexanes.

Furthermore, the compound of formula (II), wherein X is MgCl, MgBr or MgI, , may be used in form of a solution, preferably in form of a solution in one or more of the above-listed solvents. In a further preferred embodiment, the solvent used to form the solution is the only solvent present during the reaction of the compound of formula (II) with the compound of formula (III), optionally in the presence of a 1,3-diene compound and in the presence of a transition metal compound.

As described in detail above, the compound of formula (II), wherein X is MgCl, MgB, or MgI, may be prepared by a transmetallation reaction. In these cases, however, it is preferred that the compound of formula (II), wherein X is MgCl, MgBr or MgI, is provided or generated in situ in the presence of solvent preferably selected from the group consisting of THF, diethylether, dioxane.

In preferred embodiments, as already outlined above, the semifluorinated alkanes of formula (I)

F(CF₂)ₙ-(CH₂)ₘ-Rₒ (I),

wherein
- n: is an integer from 2 to 12,
- m: is 2,
- Rₒ: is a linear or branched saturated alkyl group and o depicts the number of carbon atoms,
- o: is an integer from 1 to 12, and wherein
- m+o: is an integer from 3 to 12;
are usually prepared in form of a crude reaction product. The crude reaction products or initial reaction products may or may not comprise further side-or by-products which are usually formed during the reaction in minor amounts only, if at all. As described above, after extractive workup and removal of the solvents, if used during the reaction, the crude product usually comprises the desired semifluorinated alkane of formula (I) as the major constituent. Due to the absence of isomeric and olefinic by-products, a simple distillation is sufficient to remove the side or by-products. Accordingly, the method of the present invention does not require complex fractionated distillations, which are necessary when isomeric or olefinic products are present at the end of the process, as in the prior art methods.

In preferred embodiments, however, the compounds of formula (I) as described above, preferably prepared in form of a crude reaction product, are essentially free of the compound of formula (IV)

F(CF₂)ₙ-(CH₂)ₘ-CH(CH₃)-Rₒ₋₂ (IV),

wherein
- n, m and m+o: are the same as defined in formula (I) above, and
- o: is the same as in formula (I) under the proviso that is not 1 or 2.

Preferred embodiments with respect to the values of n, m, o and m+o are the same as defined above.

In a specific preferred embodiment, the invention relates to a method of the preparation of a compound of formula (I), wherein the compound of formula (I) is F(CF₂)₄-(CH₂)₄-CH₃ (compound (Ia), F4H5), preferably as a crude reaction product and wherein the compound is essentially free of:

F(CF₂)₄-CH(CH₃)-(CH₂)₂-CH₃;

and/or

F(CF₂)₄-CH₂-CH(CH₃)-(CH₂)-CH₃;

and/or

F(CF₂)₄-(CH₂)₃-(CH₃)₂;

and/or

F(CF₂)₄-CH=CH-(CH₂)₂-CH₃;

and/or

F(CF₂)₄-CH₂-CH=CH-CH₂CH₃;

and/or

F(CF₂)₄-(CH₂)₂-CH=CH-CH₃;

and/or

F(CF₂)₄-(CH₂)₃-CH=CH₂.

Preferably, the compound is free of any of the compounds above.

In a most preferred embodiment, the invention relates to a method of the preparation of a compound of formula (I), wherein the compound is F(CF₂)₄-(CH₂)₄-CH₃ (compound (Ia), F4H5), preferably as a crude reaction product and wherein the compound is essentially free of:

F(CF₂)₄-CH(CH₃)-(CH₂)₂-CH₃; Compound (IVa).

In a further most preferred embodiment, the invention relates to a method of the preparation of a compound of formula (I), wherein the compound is F(CF₂)₄-(CH₂)₄-CH₃ (compound (Ia), F4H5), preferably as a crude reaction product and wherein the compound is essentially free of:

F(CF₂)₄-CH=CH-(CH₂)₂-CH₃ Compound (Va)

In a further most preferred embodiment, the invention relates to a method of the preparation of a compound of formula (I), wherein the compound is F(CF₂)₄-(CH₂)₄-CH₃ (compound (Ia), F4H5), preferably as a crude reaction product and wherein the compound is essentially free of any one of the following compound combinations:

F(CF₂)₄-CH(CH₃)-(CH₂)₂-CH₃ and F(CF₂)₄-CH=CH-(CH₂)₂-CH₃;

or

F(CF₂)₄-CH₂-CH(CH₃)-(CH₂)-CH₃ and F(CF₂)₄-CH₂-CH=CH-CH₂CH₃;

or

F(CF₂)₄-(CH₂)₃-(CH₃)₂ and F(CF₂)₄-(CH₂)₂-CH=CH-CH₃;

or

F(CF₂)₄-(CH₂)₃-CH=CH₂.

In a further highly preferred embodiment, the invention relates to a method of the preparation of a compound of formula (I), wherein the compound is F(CF₂)₆-(CH₂)₇-CH₃ (compound (Ib), F6H8), preferably as a crude reaction product, and wherein the compound is essentially free of:

F(CF₂)₆-CH(CH₃)-(CH₂)₅-CH₃;

and/or

F(CF₂)₆-CH₂-CH(CH₃)-(CH₂)₄-CH₃;

and/or

F(CF₂)₆-(CH₂)₂-CH(CH₃)-(CH₂)₃-CH₃;

and/or

F(CF₂)₆-(CH₂)₃-CH(CH₃)-(CH₂)₂-CH₃;

and/or

F(CF₂)₆-(CH₂)₄-CH(CH₃)-CH₂ -CH₃;

and/or

F(CF₂)₆-(CH₂)₅-CH(CH₃)₂;

and/or

F(CF₂)₆-CH=CH-(CH₂)₅-CH₃;

and/or

F(CF₂)₆-CH₂-CH=CH-(CH₂)₄-CH₃;

and/or

F(CF₂)₆-(CH₂)₂-CH=CH-(CH₂)₃-CH₃;

and/or

F(CF₂)₆-(CH₂)₃-CH=CH-(CH₂)₂-CH₃;

and/or

F(CF₂)₆-(CH₂)₄-CH=CH-(CH₂) -CH₃;

and/or

F(CF₂)₆-(CH₂)₅-CH=CH -CH₃;

and/or

F(CF₂)₆-(CH₂)₆-CH=CH₂.

In a more preferred embodiment, the compound is essentially free of any compound above.

In an alternative most preferred embodiment, the invention relates to a method of the preparation of a compound of formula (I), wherein the compound is F(CF₂)₆-(CH₂)₇-CH₃ (compound (Ib), F6H8), preferably as a crude reaction product and wherein the compound is essentially free of:

F(CF₂)₆-CH(CH₃)-(CH₂)₅-CH₃ Compound (IVb).

In an alternative most preferred embodiment, the invention relates to a method of the preparation of a compound of formula (I), wherein the compound is F(CF₂)₆-(CH₂)₇-CH₃ (compound (Ib), F6H8), preferably as a crude reaction product and wherein the compound is essentially free of:

F(CF₂)₆-CH=CH-(CH₂)₅-CH₃; Compound (Vb).

In an alternative most preferred embodiment, the invention relates to a method of the preparation of a compound of formula (I), wherein the compound is F(CF₂)₆-(CH₂)₇-CH₃ (compound (Ib), F6H8), preferably as a crude reaction product and wherein the compound is essentially free of any of the following combinations of compounds:

F(CF₂)₆-CH(CH₃)-(CH₂)₅-CH₃ and F(CF₂)₆-CH=CH-(CH₂)₅-CH₃;

or

F(CF₂)₆-CH₂-CH(CH₃)-(CH₂)₄-CH₃ and F(CF₂)₆-CH₂-CH=CH-(CH₂)₄-CH₃;

or

F(CF₂)₆-(CH₂)₂-CH(CH₃)-(CH₂)₃-CH₃ and F(CF₂)₆-(CH₂)₂-CH=CH-(CH₂)₃-CH₃;

or

F(CF₂)₆-(CH₂)₃-CH(CH₃)-(CH₂)₂-CH₃ and F(CF₂)₆-(CH₂)₃-CH=CH-(CH₂)₂-CH₃;

or

F(CF₂)₆-(CH₂)₄-CH(CH₃)-CH₂ -CH₃ and F(CF₂)₆-(CH₂)₄-CH=CH-(CH₂) -CH₃;

or

F(CF₂)₆-(CH₂)₅-CH(CH₃)₂ and F(CF₂)₆-(CH₂)₅-CH=CH -CH₃;

or

F(CF₂)₆-(CH₂)₆-CH=CH₂.

In a second aspect, which is not part of the invention and in addition to the method described above, the present disclosure relates to compounds obtainable and obtained by the invention. In particular, the present disclosure relates to compounds of formula (I), obtained or obtainable by a method according to the invention, as described above.

Also disclosed but not part of the invention are compounds of formula (I)

F(CF₂)ₙ-(CH₂)ₘ-Rₒ (I),

wherein
- n: is an integer from 2 to 12,
- m: is an integer from 0 to 7,
- Rₒ: is a linear or branched saturated alkyl group and o depicts the number of carbon atoms,
- o: is an integer from 1 to 12, wherein
- m+o: is an integer from 2 to 12;
obtained or obtainable by a method according to the method of the first aspect of the invention, as described above. In a more preferred embodiment, the invention relates to a compound of formula (I) obtained by the method of the invention as described above. Preferred embodiments with respect to the values of n, m, o and m+o are the same as defined above.

In a preferred embodiment of the disclosure, the compound of formula (I) is obtained or obtainable as crude reaction product of the method as described above, preferably the compound of formula (I) is obtained as crude reaction product of the method of the invention.

The method is in particular suitable for the preparation of linear compounds of formula (I). Accordingly, in one preferred embodiment the disclosure (not part of the invention) relates to a compound of formula (I),

F(CF₂)ₙ-(CH₂)ₘ-Rₒ (I),

wherein
- n: is an integer from 2 to 12,
- m: is an integer from 0 to 7,
- Rₒ: is a linear saturated alkyl group and o depicts the number of carbon atoms,
- o: is an integer from 1 to 12, wherein
- m+o: is an integer from 2 to 12;
obtained or obtainable by a method according to the method of the invention, preferably as a crude reaction product of the method, more preferably the compound is obtained by the method of the invention, most preferably the compound is obtained as a crude reaction product by the method of the invention. Preferred embodiments with respect to the values of n, m, o and m+o are the same as defined above.

As discussed before, an advantage of the method of the invention is the absence of undesired side products, in particular undesired branched compounds or olefinic compounds.

As such, in one embodiment, the disclosure relates to a compound of formula (I) as defined above obtainable or obtained by the method of the invention, wherein
- Rₒ: is a linear saturated alkyl group and o depicts the number of carbon atoms,
and, wherein
the compound is essentially free of the compound of formula (IV)

F(CF₂)ₙ-(CH₂)ₘ-CH(CH₃)-Rₒ₋₂ (IV),

wherein
- n, m and m+o: are the same as defined in formula (I) above, and
- o: is the same as in formula (I) with the proviso that is not 1 or 2.

In a particular embodiment, the disclosure relates to a compound of formula (I) as defined above obtainable or obtained by the method of the invention, wherein
- Rₒ: is a linear saturated alkyl group and o depicts the number of carbon atoms,
and, wherein
the compound is essentially free of the compound of formula (IV)

F(CF₂)ₙ-(CH₂)ₘ-CH(CH₃)-Rₒ₋₂ (IV),

wherein
n, m and m+o are the same as defined in formula (I) above, and
o is an integer from 3 to 12. In a further preferred embodiment, the invention relates to a compound of formula (I) as defined above obtainable or obtained by the method of the invention, wherein

- Rₒ: is a linear saturated alkyl group and o depicts the number of carbon atoms,
and,
wherein the compound is essentially free of the compound of formula (V)

F(CF₂)ₙ-(CH₂)ₘ-CH=CH-Rₒ₋₂ (V),

wherein
n, m and m+o are the same as defined in formula (I), and
o is the same as in formula (I) with the proviso that is not 1 or 2.

In a more preferred embodiment, the disclosure relates to a compound as defined above obtainable or obtained by the method of the invention, wherein
- Rₒ: is a linear saturated alkyl group and o depicts the number of carbon atoms,
and, wherein
wherein the compound is essentially free of the compound of formula (V)

F(CF₂)ₙ-(CH₂)ₘ-CH=CH-Rₒ₋₂ (V),

wherein
n, m and m+o are the same as defined in formula (I), and
o is an integer from 3 to 12.

In a more preferred embodiment of the disclosure (not part of the invention), the disclosure relates to a compound of formula (I) as defined above obtainable or obtained by the method of the invention, wherein the compound is essentially free of the compound of formula (IV) as defined above and essentially free of the compound of formula (V) as defined above.

Preferred embodiments with respect to the values of n, m, o and m+o are the same as defined previously.

The disclosure relates in particular to compounds (Ia) and (Ib) as defined above, wherein the compounds are essentially free of branched or olefinic compounds.

In a specific embodiment, the disclosure (not part of the invention) relates to a compound of formula (I), wherein the compound is F(CF₂)₄-(CH₂)₄-CH₃ (compound (Ia), F4H5), wherein the compound is obtained or obtainable by the method of the invention, preferably as a crude reaction product and wherein the compound is essentially free of:

F(CF₂)₄-CH(CH₃)-(CH₂)₂-CH₃;

and/or

F(CF₂)₄-CH₂-CH(CH₃)-(CH₂)-CH₃;

and/or

F(CF₂)₄- -(CH₂)₃-(CH₃)₂;

and/or

F(CF₂)₄-CH=CH-(CH₂)₂-CH₃;

and/or

F(CF₂)₄-CH₂-CH=CH-CH₂CH₃;

and/or

F(CF₂)₄-(CH₂)₂-CH=CH-CH₃;

and/or

F(CF₂)₄-(CH₂)₃-CH=CH₂.

More preferably, the compound is free of any the above compounds.

In a most preferred embodiment, the disclosure relates to a compound of formula (I), wherein the compound is F(CF₂)₄-(CH₂)₄-CH₃ (compound (Ia), F4H5), wherein the compound is obtained or obtainable by the method of the invention, preferably as a crude reaction product and wherein the compound is essentially free of:

F(CF₂)₄-CH(CH₃)-(CH₂)₂-CH₃ Compound (IVa).

In a further most preferred embodiment, the disclosure relates to a compound of formula (I), wherein the compound is F(CF₂)₄-(CH₂)₄-CH₃ (compound (Ia), F4H5), wherein the compound is obtained or obtainable by the method of the invention, preferably as a crude reaction product and wherein the compound is essentially free of:

F(CF₂)₄-CH=CH-(CH₂)₂-CH₃ Compound (Va)

In a particular preferred embodiment, the disclosure relates to a compound of formula (I), wherein the compound is F(CF₂)₄-(CH₂)₄-CH₃ (compound (Ia), F4H5), wherein the compound is obtained or obtainable by the method of the invention, preferably as a crude reaction product and wherein the compound is essentially free of any one of the following combinations of compounds:

F(CF₂)₄-CH(CH₃)-(CH₂)₂-CH₃ and F(CF₂)₄-CH=CH-(CH₂)₂-CH₃;

or

F(CF₂)₄-CH₂-CH(CH₃)-(CH₂)-CH₃ and F(CF₂)₄-CH₂-CH=CH-CH₂CH₃;

or

F(CF₂)₄- -(CH₂)₃-(CH₃)₂ and F(CF₂)₄-(CH₂)₂-CH=CH-CH₃;

or

F(CF₂)₄-(CH₂)₃-CH=CH₂.

In a further preferred embodiment, the disclosure relates to a compound of formula (I), wherein the compound is F(CF₂)₆-(CH₂)₇-CH₃ (compound (Ib), F6H8), wherein the compound is obtained or obtainable by the method of the invention, preferably as a crude reaction product and wherein the compound is essentially free of:

F(CF₂)₆-CH(CH₃)-(CH₂)₅-CH₃;

and/or

F(CF₂)₆-CH₂-CH(CH₃)-(CH₂)₄-CH₃;

and/or

F(CF₂)₆-(CH₂)₂-CH(CH₃)-(CH₂)₃-CH₃;

and/or

F(CF₂)₆-(CH₂)₃-CH(CH₃)-(CH₂)₂-CH₃;

and/or

F(CF₂)₆-(CH₂)₄-CH(CH₃)-CH₂ -CH₃;

and/or

F(CF₂)₆-(CH₂)₅-CH(CH₃)₂;

and/or

F(CF₂)₆-CH=CH-(CH₂)₅-CH₃;

and/or

F(CF₂)₆-CH₂-CH=CH-(CH₂)₄-CH₃;

and/or

F(CF₂)₆-(CH₂)₂-CH=CH-(CH₂)₃-CH₃;

and/or

F(CF₂)₆-(CH₂)₃-CH=CH-(CH₂)₂-CH₃;

and/or

F(CF₂)₆-(CH₂)₄-CH=CH-(CH₂)-CH₃;

and/or

F(CF₂)₆-(CH₂)₅-CH=CH-CH₃;

and/or

F(CF₂)₆-(CH₂)₆-CH=CH₂.

Preferably, the compound is free of any compound above.

In a most preferred embodiment, the disclosure relates to a compound of formula (I), wherein the compound is F(CF₂)₆-(CH₂)₇-CH₃ (compound (Ib), F6H8), wherein the compound is obtained or obtainable by the method of the invention, preferably as a crude reaction product and wherein the compound is essentially free of:

F(CF₂)₆-CH(CH₃)-(CH₂)₅-CH₃; Compound (IVb).

In a most preferred embodiment, the disclosure relates to a compound of formula (I), wherein the compound is F(CF₂)₆-(CH₂)₇-CH₃ (compound (Ib), F6H8), wherein the compound is obtained or obtainable by the method of the invention, preferably as a crude reaction product and wherein the compound is essentially free of:

F(CF₂)₆-CH=CH-(CH₂)₅-CH₃ Compound (Vb).

In a most preferred embodiment, the disclosure relates to a compound of formula (I), wherein the compound is F(CF₂)₆-(CH₂)₇-CH₃ (compound (Ib), F6H8), wherein the compound is obtained or obtainable by the method of the invention, preferably as a crude reaction product and wherein the compound is essentially free of any one of the following combinations of compounds:

F(CF₂)₆-CH(CH₃)-(CH₂)₅-CH₃ and F(CF₂)₆-CH=CH-(CH₂)₅-CH₃;

or

F(CF₂)₆-CH₂-CH(CH₃)-(CH₂)₄-CH₃ and F(CF₂)₆-CH₂-CH=CH-(CH₂)₄-CH₃;

or

F(CF₂)₆-(CH₂)₂-CH(CH₃)-(CH₂)₃-CH₃ and F(CF₂)₆-(CH₂)₂-CH=CH-(CH₂)₃-CH₃;

or

F(CF₂)₆-(CH₂)₃-CH(CH₃)-(CH₂)₂-CH₃ and F(CF₂)₆-(CH₂)₃-CH=CH-(CH₂)₂-CH₃;

or

F(CF₂)₆-(CH₂)₄-CH(CH₃)-CH₂ -CH₃ and F(CF₂)₆-(CH₂)₄-CH=CH-CH₂-CH₃;

or

F(CF₂)₆-(CH₂)₅-CH(CH₃)₂ and F(CF₂)₆-(CH₂)₅-CH=CH -CH₃;

or

F(CF₂)₆-(CH₂)₆-CH=CH₂.

It is preferred that the compounds (Ia) and (Ib) are obtained as crude reaction products by the method of the invention and essentially free of the compounds as defined above.

Also disclosed, but not part of the invention, is a composition comprising a compound of formula (I), wherein the compound of formula (I) is obtained or obtainable by a method according to the invention, preferably as a crude reaction product.

In a particular embodiment, the disclosure relates to a composition comprising a compound of formula (I)

F(CF₂)ₙ-(CH₂)ₘ-Rₒ (I),

wherein
- n: is an integer from 2 to 12,
- m: is an integer from 0 to 7,
- Rₒ: is a linear or branched saturated alkyl group and o depicts the number of carbon atoms,
- o: is an integer from 1 to 12, wherein
- m+o: is an integer from 2 to 12;
wherein the compound is obtained or obtainable by a method of the invention as described above. In a preferred embodiment of the invention, the compound of formula (I) is obtained as a crude reaction product.

Preferred embodiments with respect to the values of n, m, o and m+o are the same as defined above.

Also disclosed, but not part of the invention, is a composition comprising a compound of formula (I)

F(CF₂)ₙ-(CH₂)ₘ-Rₒ (I),

wherein
- n: is an integer from 2 to 12, preferably 3 to 9, more preferably 4, 5 or 6
- m: is an integer from 0 to 7,
- Rₒ: is a linear saturated alkyl group and o depicts the number of carbon atoms,
- o: is an integer from 1 to 12, wherein
- m+o: is an integer from 2 to 12; preferably 2 to 9 or 3 to 10, more preferably 5, 6, 7 or 8;
wherein the compound is obtained or obtainable by a method according to the invention. In a preferred embodiment of the disclosure, the compound of formula (I) as defined above is obtained as a crude reaction product.

Preferred embodiments with respect to the values of n, m, o and m+o are the same as defined above.

In a more preferred embodiment, the disclosure relates to a composition comprising a compound of formula (I),

F(CF₂)ₙ-(CH₂)ₘ-Rₒ (I),

wherein
- n: is an integer from 2 to 12, preferably 3 to 9, more preferably 4, 5 or6
- m: is an integer from 0 to 7,
- Rₒ: is a linear saturated alkyl group and o depicts the number of carbon atoms,
- o: is an integer from 1 to 12, wherein
- m+o: is an integer from 2 to 12; preferably 2 to 9 or3 to 10, more preferably 5,6,7 or 8;
wherein the compound is obtained or obtainable by a method according to the invention; and wherein the composition is substantially free of the compound of formula (IV)

F(CF₂)ₙ-(CH₂)ₘ-CH(CH₃)-Rₒ₋₂ (IV),

wherein
n, m and m+o are as defined in formula (I), and
o is as defined in formula (I) with the proviso that is not 1 or 2.

In a preferred embodiment of the disclosure, the composition comprises a compound of formula (I) as defined above and is substantially free of the compound of formula (IV) as defined above, wherein
- o: is an integer from 3 to 12.

Preferred embodiments with respect to the values of n, m, o and m+o are the same as defined previously.

In a further preferred embodiment, the disclosure relates to a composition comprising a compound of formula (I),

F(CF₂)ₙ-(CH₂)ₘ-Rₒ (I),

wherein
- n: is an integer from 2 to 12, preferably 3 to 9, more preferably 4, 5 or6
- m: is an integer from 0 to 7,
- Rₒ: is a linear saturated alkyl group and o depicts the number of carbon atoms,
- o: is an integer from 1 to 12, wherein
- m+o: is an integer from 2 to 12; preferably 3 to 10, more preferably 5, 6, 7 or 8;
wherein the compound is obtained or obtainable by a method according to the invention; and wherein the composition is substantially free of the compound of formula (V)

F(CF₂)ₙ-(CH₂)ₘ-CH=CH-Rₒ₋₂ (V),

wherein
n, m and m+o are as defined in formula (I), and
o is as defined in formula (I) with the proviso that is not 1 or 2.

In a preferred embodiment of the disclosure, the composition comprises a compound of formula (I) as defined above and is substantially free of the compound of formula (V) as defined above, wherein
- o: is an integer from 3 to 12.

In an even more preferred embodiment of the disclosure, the disclosure relates to a composition comprising a compound of formula (I),

F(CF₂)ₙ-(CH₂)ₘ-Rₒ (I),

wherein
- n: is an integer from 2 to 12, preferably 3 to 9, more preferably 4, 5 or 6
- m: is an integer from 0 to 7,
- Rₒ: is a linear saturated alkyl group and o depicts the number of carbon atoms,
- o: is an integer from 1 to 12, wherein
- m+o: is an integer from 2 to 12; preferably 3 to 10, more preferably 5, 6, 7 or 8;
wherein the compound is obtained or obtainable by a method according to the invention; and wherein the composition is substantially free of the compound of formula (IV)

F(CF₂)ₙ-(CH₂)ₘ-CH(CH₃)-Rₒ₋₂ (IV),

wherein
n, m and m+o are as defined in formula (I), and
o is as defined in formula (I) with the proviso that is not 1 or 2, preferably o is an integer from 3 to 12; and
wherein the composition is substantially free of the compound of formula (V)

   F(CF₂)ₙ-(CH₂)ₘ-CH=CH-Rₒ₋₂ (V),
wherein
n, m and m+o are as defined in formula (I), and
o is as defined in formula (I) with the proviso that is not 1 or 2, preferably o is an inter from 3 to 12.

In a specific preferred embodiment of the disclosure (not part of the invention), the disclosure relates to a composition comprising a compound of formula (I), wherein the compound of formula (I) is F(CF₂)₄-(CH₂)₄-CH₃ (compound (Ia)), F4H5), wherein compound (Ia) is obtainable or obtained by the method according to the invention, optionally as a crude reaction product. Preferably, the compound (Ia) F(CF₂)₄-(CH₂)₄-CH₃ is obtained by the method of the invention, more preferably, the compound F(CF₂)₄-(CH₂)₄-CH₃ is obtained by the method of the invention as a crude reaction product.

In a more preferred embodiment, the disclosure relates to a composition comprising a compound of formula (I), wherein the compound of formula (I) is F(CF₂)₄-(CH₂)₄-CH₃ (compound (Ia)), F4H5), wherein compound (Ia) is obtainable or obtained by the method according to the invention, preferably as a crude reaction product and wherein the composition is essentially free of

F(CF₂)₄-CH(CH₃)-(CH₂)₂-CH₃;

and/or

F(CF₂)₄-CH₂-CH(CH₃)-CH₂-CH₃;

and/or

F(CF₂)₄- -(CH₂)₃-(CH₃)₂;

and/or

F(CF₂)₄-CH=CH-(CH₂)₂-CH₃;

and/or

F(CF₂)₄-CH₂-CH=CH-CH₂-CH₃;

and/or

F(CF₂)₄-(CH₂)₂-CH=CH-CH₃;

and/or

F(CF₂)₄-(CH₂)₃-CH=CH₂.

Preferably, the composition is free of any of said compounds.

In an even more preferred embodiment, the disclosure relates to a composition comprising a compound of formula (I), wherein the compound of formula (I) is F(CF₂)₄-(CH₂)₄-CH₃ (compound (Ia), F4H5), wherein compound (Ia) is obtainable or obtained by the method according to the invention, preferably as a crude reaction product and wherein the composition is essentially free of

F(CF₂)₄-CH(CH₃)-(CH₂)₂-CH₃ Compound (IVa).

In an even more preferred embodiment, the disclosure relates to a composition comprising a compound of formula (I), wherein the compound of formula (I) is F(CF₂)₄-(CH₂)₄-CH₃ (compound (Ia), F4H5), wherein compound (Ia) is obtainable or obtained by the method according to the invention, preferably as a crude reaction product and wherein the composition is essentially free of

F(CF₂)₄-CH=CH-(CH₂)₂-CH₃ Compound (Va).

In particular the composition comprising a compound of formula (I), wherein the compound of formula (I) is F(CF₂)₄-(CH₂)₄-CH₃ (compound (Ia), F4H5), wherein compound (Ia) is obtainable or obtained by the method according to the invention is free of any of the following combinations of compounds:

F(CF₂)₄-CH(CH₃)-(CH₂)₂-CH₃ and F(CF₂)₄-CH=CH-(CH₂)₂-CH₃;

or

F(CF₂)₄-CH₂-CH(CH₃)-CH₂-CH₃ and F(CF₂)₄-CH₂-CH=CH-CH₂-CH₃;

or

F(CF₂)₄- -(CH₂)₃-(CH₃)₂ and F(CF₂)₄-(CH₂)₂-CH=CH-CH₃;

or

F(CF₂)₄-(CH₂)₃-CH=CH₂.

In a further specific preferred embodiment of the disclosure, the disclosure relates to a composition comprising a compound of formula (I), wherein the compound of formula (I) is F(CF₂)₆-(CH₂)₇-CH₃ (compound (Ib), F6H8), wherein compound (Ib) is obtainable or obtained by the method according to the invention, optionally as a crude reaction product. Preferably, the compound (Ib) F(CF₂)₆-(CH₂)₇-CH₃ is obtained by the method of the invention, more preferably, the compound F(CF₂)₆-(CH₂)₇-CH₃ is obtained by the method of the invention as a crude reaction product.

In a more preferred embodiment, the disclosure relates to a composition comprising a compound of formula (I), wherein the compound of formula (I) is F(CF₂)₆-(CH₂)₇-CH₃ (compound (Ib), F6H8), wherein compound (Ib) is obtainable or obtained by the method according to the invention, preferably as a crude reaction product and wherein the composition is essentially free of

F(CF₂)₆-CH(CH₃)-(CH₂)₅-CH₃;

and/or

F(CF₂)₆-CH₂-CH(CH₃)-(CH₂)₄-CH₃;

and/or

F(CF₂)₆-(CH₂)₂-CH(CH₃)-(CH₂)₃-CH₃;

and/or

F(CF₂)₆-(CH₂)₃-CH(CH₃)-(CH₂)₂-CH₃;

and/or

F(CF₂)₆-(CH₂)₄-CH(CH₃)-CH₂ -CH₃;

and/or

F(CF₂)₆-(CH₂)₅-CH(CH₃)₂;

and/or

F(CF₂)₆-CH=CH-(CH₂)₅-CH₃;

and/or

F(CF₂)₆-CH₂-CH=CH-(CH₂)₄-CH₃;

and/or

F(CF₂)₆-(CH₂)₂-CH=CH-(CH₂)₃-CH₃;

and/or

F(CF₂)₆-(CH₂)₃-CH=CH-(CH₂)₂-CH₃;

and/or

F(CF₂)₆-(CH₂)₄-CH=CH-(CH₂) -CH₃;

and/or

F(CF₂)₆-(CH₂)₅-CH=CH -CH₃;

and/or

F(CF₂)₆-(CH₂)₆-CH=CH₂.

Preferably, the composition is free of any compound noted above.

In a more preferred embodiment of the disclosure, the disclosure relates to a composition comprising a compound of formula (I), wherein the compound of formula (I) is F(CF₂)₆-(CH₂)₇-CH₃ (compound (Ib), F6H8), wherein compound (Ib) is obtainable or obtained by the method according to the invention, preferably as a crude reaction product and wherein the composition is essentially free of

F(CF₂)₆-CH(CH₃)-(CH₂)₅-CH₃ Compound (IVb).

In a more preferred embodiment of the disclosure, the disclosure relates to a composition comprising a compound of formula (I), wherein the compound of formula (I) is F(CF₂)₆-(CH₂)₇-CH₃ (compound (Ib), F6H8), wherein compound (Ib) is obtainable or obtained by the method according to the invention, preferably as a crude reaction product and wherein the composition is essentially free of

F(CF₂)₆-CH=CH-(CH₂)₅-CH₃ Compound (Vb).

In particular the composition comprising a compound of formula (I), wherein the compound of formula (I) is F(CF₂)₆-(CH₂)₇-CH₃ (compound (Ib), F6H8), wherein compound (Ia) is obtainable or obtained by the method according to the invention is free of any of the following combinations of compounds:

F(CF₂)₆-CH(CH₃)-(CH₂)₅-CH₃ and F(CF₂)₆-CH=CH-(CH₂)₅-CH₃;

or

F(CF₂)₆-CH₂-CH(CH₃)-(CH₂)₄-CH₃ and F(CF₂)₆-CH₂-CH=CH-(CH₂)₄-CH₃;

or

F(CF₂)₆-(CH₂)₂-CH(CH₃)-(CH₂)₃-CH₃ and F(CF₂)₆-(CH₂)₂-CH=CH-(CH₂)₃-CH₃;

or

F(CF₂)₆-(CH₂)₃-CH(CH₃)-(CH₂)₂-CH₃ and F(CF₂)₆-(CH₂)₃-CH=CH-(CH₂)₂-CH₃;

or

F(CF₂)₆-(CH₂)₄-CH(CH₃)-CH₂ -CH₃ and F(CF₂)₆-(CH₂)₄-CH=CH-CH₂-CH₃;

or

F(CF₂)₆-(CH₂)₅-CH(CH₃)₂ and F(CF₂)₆-(CH₂)₅-CH=CH-CH₃;

or

F(CF₂)₆-(CH₂)₆-CH=CH₂.

In a particularly preferred embodiment, the disclosure relates to a composition comprising a compound of formula (I) obtainable or obtained by the method of the invention preferably as a crude reaction product, wherein the compound is compound (Ia)

F(CF₂)₄-(CH₂)₄-CH₃ (Ia)

or the compound (Ib)

F(CF₂)₆-(CH₂)₇-CH₃ (Ib);

wherein the composition substantially consists of the compound (Ia) or (Ib).

In some embodiments of the disclosure, the composition comprising a compound of formula (I) obtained or obtainable by the method of the invention additionally comprises a pharmaceutically active compound.

The composition comprising a compound according to formula (I), obtained or obtainable by the method of the present invention might be any suitable composition. Preferably, the composition is formulated as a liquid composition, more preferably as a clear liquid composition.

In particular, it is preferred that the composition comprising a compound of formula (I) obtained or obtainable by the method of the invention is further comprising an active pharmaceutical ingredient.

In this context, clear means the absence of dispersed solid or liquid particles which cause turbidity. In other words, such clear solution is a purely monophasic liquid system, except that minor and technically irrelevant amounts of particulate impurities may be present.

In optional embodiments, the compositions, when comprising an active pharmaceutical ingredient or any excipients, may be formulated to be administered as a gel, suspension, microemulsion, emulsion or a spray. In a preferred embodiment, said composition is in form of a solution, suspension or emulsion. Preferably, the compositions are provided in sterile form.

The pharmaceutical composition comprising a compound of formula (I) obtainable or obtained by the method of the invention may be provided in form of a suspension. A suspension may be defined as a type of a dispersion, a dispersion being a system having at least one continuous (or coherent) phase and at least one discontinuous (or inner) phase which is dispersed in the continuous phase. In a suspension, the dispersed phase is in the solid state. The suspensions useful for practising the present disclosure are liquids, at least at physiological temperature, which means that the continuous phase is a liquid. Typically, the suspensions are also liquid at room temperature.

In a particularly preferred embodiment, compositions comprising a compound of formula (I) obtainable or obtained by the method of the invention is in a liquid form and comprises at least 80 wt.-% or even 90 wt.-% of the compound, in particular from 90 wt.-% to 99.9 wt.-% or even 100 wt.-%, based on the total weight of the composition. In other embodiments, the composition may comprise from about 95 wt.-% to about 99.95 wt.-%, or from about 97 wt.-% to about 99.9 wt.-%, or from about 98 wt.-% to about 99.5 wt.-% of the compound based on the total weight of the composition.

The compositions as defined above may also comprise further excipients as required or as useful, such as one or more acids, bases, electrolytes, buffers, solutes, antioxidants, stabilizers, and if required, preservatives. In one preferred embodiment, the compositions as defined above are substantially free of water and/or substantially free of a preservative.

In some embodiments of the disclosure, the composition of the present disclosure additionally may comprise one or more lipophilic liquid constituents. The optional lipophilic liquid constituents are preferably substantially non-water soluble and/or non-water miscible excipients, for example oily excipient such as lipids, triglyceride oils and any other oils that are physiologically tolerated, preferably physiologically tolerated by the eye.

In some embodiments of the disclosure, the present compositions comprising a compound of formula (I) obtained or obtainable by the method of the invention comprises a surfactant. The surfactant may be ionic or non-ionic. In a particular embodiment, the composition comprises an ionic surfactant preferably selected from the class of phospholipids. Phospholipids are surfactants composed of a phosphate group (imparting a negative charge) which is, on one side, linked to a small basic residue (usually imparting a positive charge) such as choline or ethanolamine, and on the other side to glycerol or sphingosine. The glycerol residue is esterified with two fatty acid residues which represent the lipophilic part of most of the phospholipid molecules.

Among the preferred phospholipids are native, hydrated and/or purified lecithins, such as lecithin derived from eggs or soy beans, typically comprising high amounts of phosphatidylcholines. Also preferred are native, purified, synthetic or semisynthetic phosphatidylcholines, either having mixed fatty acid residues as found in their native sources or generated by hydration; or specific fatty acid compositions as in the case of e.g. dimyristoyl phosphatidylcholine, dipalmitoyl phosphatidylcholine, and distearoyl phosphatidylcholine. Furthermore, phospholipid extracted from animal organs such as lungs may be used, for example pulmonary phospholipids from pigs, as comprised in the product, Curosurf^{®}. Particularly preferred surfactants are purified and optionally hydrated lecithins extracted from eggs or soy beans.

In an alternative preferred embodiment, the surfactant is selected from the class of physiologically acceptable nonionic surfactants. Examples for such surfactants include in particular:
- pegylated glycerides such as macrogol-15-hydroxystearate (e.g. Solutol^{®} HS 15), macrogol glycerol ricinoleate-35 (e.g. Cremophor^{®} EL), macrogol glycerol hydroxystearate-40 (e.g. Cremophor^{®} RH 40), macrogol-1000-glycerol monolaurate, macrogol-1000-glycerol monostearate, and macrogol-1000-glycerol monooleate;
- pegylated fatty acids such as macrogol stearate 400, polyoxyl 40 stearate, and polyoxyl 60 stearate;
- pegylated fatty alcohols such as macrogol laurylether, polyoxyl 20 cetostearylether, and polyoxyl 10 oleylether;
- pegylated sorbitan fatty acid esters such as polysorbate 20, polysorbate 40, polysorbate 60, and polysorbate 80 (e.g. Tween^{®} 20/40/60/80); and
- triblock copolymers of polyoxyethylene and polyoxypropylene, such as poloxamer 124, poloxamer 188, poloxamer 237, poloxamer 338, and poloxamer 407. From the above, polysorbates are particularly preferred.

It is also possible to incorporate more than one surfactant within the composition comprising a compound of formula (I) obtained or obtainable by the method of the invention. For example, the combination of an ionic surfactant such as a lecithin and a nonionic surfactant such as a polysorbate or poloxamer may be useful for stabilising the emulsion in case that the continuous phase comprises substantial amounts of salts or other electrolytes.

The composition comprising a compound of formula (I) obtained or obtainable by the method of the invention may further comprise excipients in range of up to about 10% (w/v), more preferably up to about 5% (w/v), even more preferably up to about 2% (w/v). Accordingly, in a preferred embodiment the composition comprises a pharmaceutically acceptable excipient.

The term "excipients" as used herein refers to any pharmaceutically acceptable natural or synthetic substance that may be added to the pharmaceutical compositions of the present disclosure to enhance or otherwise modify its physical or chemical constitution or stability or therapeutic properties. The compositions comprising a compound of formula (I) obtained or obtainable by the method of the invention may optionally comprise one or more excipients such as, for example, an antioxidant, a preservative, a lipid or oily excipient, a surfactant or a lubricant or a combination of at least 2 excipients thereof.

Suitable antioxidants for use in the compositions comprising a compound of formula (I) obtained or obtainable by the method of the invention comprise, for example: butylated hydroxytoluene (BHT), butylated hydroxyanisole (BHA), tertiary butylhydroquinone (TBHQ), vitamin E, vitamin E derivatives (i.e. alpha-tocopherol acetate) and/or ascorbic acid.

Further suitable excipients include, but are not limited to, oil selected from glyceride oils, liquid waxes, and liquid paraffin, or an organic solvent exhibiting a high degree of biocompatibility, or a mixture of more than one liquid excipients.

Examples of potentially useful oily excipients which may be used in combination with one or more SFA's as described above may include triglyceride oils (i.e. soybean oil, olive oil, sesame oil, cotton seed oil, castor oil, sweet almond oil), mineral oil (i.e. petrolatum and liquid paraffin), medium chain triglycerides (MCT), oily fatty acids, isopropyl myristate, oily fatty alcohols, esters of sorbitol and fatty acids, oily sucrose esters, or any other oily substance which is physiologically tolerated, preferably physiologically tolerated by the eye.

Suitable lubricants for use in the present disclosure comprise, for example, carboxymethylcellulose and its sodium salt (CMC, carmellose), polyvinyl alcohol, hydroxypropyl methylcellulose (HPMC, hypromellose), hyaluronic acid and its sodium salt, and hydroxypropyl guar gum.

Examples of potentially useful organic solvents include glycerol, propylene glycol, polyethylene glycol, and ethanol. However, the concentration of the cosolvent should preferably be low relative to that of the SFA or SFA mixture. If an organic solvent such as ethanol is used, it is recommendable to keep it below a level of approx. 5 wt.-%. More preferably, the content of ethanol is from about 0.1 to about 2 wt.-%, and most preferably not more than about 1 wt.-%. In one preferred embodiment, the suspension compositions according to the disclosure are free of an organic liquid or solvent. In a particularly preferred embodiment, the suspensions are free of ethanol.

The compositions comprising a compound of formula (I) obtainable or obtained by the method of the invention may comprise further pharmaceutical excipients as required or useful. Potentially useful excipients include acids, bases, antioxidants, stabilisers, synergists, coloring agents and thickening agents. In a preferred embodiment, however, the liquid vehicle of the pharmaceutical composition according to the present disclosure is free of any (further) excipients.

The compositions comprising a compound of formula (I) obtainable or obtained by the method of the present invention may comprise a pharmaceutically acceptable preservative, in a preferred embodiment, the pharmaceutical compositions of the present embodiment are free of a preservative.

In a further embodiment, water can also be present in the composition comprising a compound of formula (I) obtainable or obtained by the method of the present invention, however, preferably in small amounts of up 1.0 wt.-% or even up to 0.1 wt.-% or less, based on the final composition (final dosage form). In a preferred embodiment, the composition comprising a compound of formula (I) obtainable or obtained by the method of the present invention is essentially free of water.

In a most preferred embodiment of the disclosure, the composition comprising a compound of formula (I) obtainable or obtained by the method of the invention is formulated as an ophthalmic composition.

Also disclosed, but not part of the invention, are compounds according to the second aspect of the disclosure and/or the compositions according to the third aspect of the disclosure for use as a medicine. Accordingly, in this aspect, the present disclosure relates to compounds of formula (I), obtained or obtainable by a method according to the invention, as described above, for use as a medicine.

More specifically, in this aspect the disclosure provides compounds of formula (I)

F(CF₂)ₙ-(CH₂)ₘ-Rₒ (I),

wherein
- n: is an integer from 2 to 12,
- m: is an integer from 0 to 7,
- Rₒ: is a linear or branched saturated alkyl group and o depicts the number of carbon atoms,
- o: is an integer from 1 to 12, wherein
- m+o: is an integer from 2 to 12;
obtained or obtainable by a method according to the method of the first aspect of the invention, for use as a medicine. In addition to this or alternatively, in this aspect the present disclosure provides the composition according to the third aspect of the disclosure comprising a compound of formula (I), wherein the compound of formula (I) is obtained or obtainable by a method according to the invention, preferably as a crude reaction product, for use as a medicine. In a more preferred embodiment, this aspect of the disclosure relates to a compound of formula (I) obtained by the method of the invention as described above, for use as a medicine.

In a particular embodiment, in this aspect, the disclosure provides a composition comprising a compound of formula (I)

F(CF₂)ₙ-(CH₂)ₘ-Rₒ (I),

wherein
- n: is an integer from 2 to 12,
- m: is an integer from 0 to 7,
- Rₒ: is a linear or branched saturated alkyl group and o depicts the number of carbon atoms,
- o: is an integer from 1 to 12, wherein
- m+o: is an integer from 2 to 12;
wherein the compound is obtained or obtainable by a method of the invention as described above, for use as a medicine. In a preferred embodiment of this aspect of the disclosure, the compound of formula (I) for use as a medicine is obtained as a crude reaction product.

The compounds of formula (I) as well as the compositions comprising a compound of formula (I) including all embodiments thereof as described above for the first aspect of the invention are especially useful for the prevention and/or treatment of a disease or condition affecting a tissue related to the eye and/or the skin and/or the ear and/or the lung and/or the nose of a subject, preferably of a human subject.

Accordingly, in a fifth aspect, the present disclosure relates to the compounds of formula (I) as described for the second aspect of the disclosure and/or to the compositions comprising a compound of formula (I) as described for the third aspect of the disclosure for use in the prevention and/or treatment of a disease or condition affecting a tissue related to the eye and/or the skin and/or the ear and/or the lung and/or the nose of a subject, preferably a human subject.

Accordingly, the compounds and/or compositions of the present disclosure are useful as pharmaceutical compounds and/or compositions. In some embodiments, the compounds of formula (I) as described above or the composition comprising a compound of formula (I) are especially useful as an ophthalmic compound or composition, and may preferably be administered topically to the eye, eye lid, eye sac, eye surface and/or to an ophthalmic tissue of a patient. Preferably, the pharmaceutical compound or composition of this aspect of the present disclosure may be topically administered to an outer surface of an eye of a patient or to an ophthalmic tissue which is readily accessible by the patient or by another person administering the pharmaceutical compound or composition to the eye of the patient in need thereof.

In further embodiments, the compounds of formula (I) or the compositions comprising such compounds may be useful for the prevention and/or treatment of a disease or condition affecting a tissue related to the skin, to the ear and/or to the nose of a patient. In these cases, the compound or compositions may be applied to the affected tissue topically and/or by injection, preferably, however, by topical application.

In other embodiments, the compounds of formula (I) or the compositions comprising such compounds may be useful for the prevention and/or treatment of a disease or condition affecting a tissue related to the lung of a subject or patient. In this case, the compound or compositions may, for example, be applied to the affected tissue by inhalation.

The compounds or composition for use according to this aspect of the disclosure may be used for the treatment of subjects, preferably human subjects or patients of any age including infants, children, adults and elderly adults. Accordingly, in some embodiments, the compounds or compositions according to this aspect of the disclosure may be used in pediatric or adult treatment.

The present compounds of formula (I) or the corresponding pharmaceutical compositions comprising a compound of formula (I), especially when used as liquid of either low or higher viscosity (usually in the range of 1 to 3.5 mPa s) may advantageously be administered in form of drops or by spraying or by injection. Most preferably, however, the liquid pharmaceutical composition of the present disclosure may be administered as drops, more specifically as eyedrops to be administered topically to the eye.

Depending on the extent of the disease, or whether or not both eyes of the patient to be treated are affected, the drops or eyedrops of the present ophthalmic pharmaceutical compounds or compositions may be administered to only one eye or to both eyes of the patient. The present pharmaceutical compounds or compositions, provides droplet sizes when administered from conventional droppers, with a volume usually in the range from about 5 to about 15 µl. This small droplet size usually facilitates the dropwise administration and, moreover, facilitates precise dosage of the pharmaceutical composition of the present disclosure. Accordingly, the ophthalmic pharmaceutical composition of the present disclosure is administered as single drops with a volume of about 5 to 15 µl per dose per eye, preferably with a volume of about 8 to 13 µl per dose per eye, more preferably with a volume of about 9 to 12 µl per dose per eye and most preferably with a volume of about 10 to 11 µl per dose per eye.

In some preferred embodiments, the compounds of formula (I) obtained or obtainable by a method according to the first aspect of the invention or the compositions comprising such a compound may be useful in the treatment for the treatments of ophthalmic disease or conditions such as, for example, keratoconjunctivitis sicca (dye eye disease), meibomian gland dysfunction and/or blepharitis.

In other preferred embodiments, the compounds of formula (I) obtained or obtainable by a method according to the first aspect of the invention or the compositions comprising such a compound may be useful in the treatment for the treatments of diseases or conditions affecting the skin, such as, for example psoriasis, dermatitis, erythema, eczema, allergy, acne, actinic keratosis, fungal infections, bacterial infections, viral infections, verrucae, or rosacea.

Also disclosed, but not part of the invention, is a method for the treatment and/or prevention of a disease or condition affecting a tissue related to the eye and/or the skin and/or the ear and/or the lung and/or the nose of a subject, comprising administering the compound of formula (I) obtained or obtainable by a method according to the first aspect of the invention or the compositions comprising such a compound to the affected tissue.

It should be noted that also for the method according to this sixths aspect of the disclosure all embodiments and preferred embodiments as described above in connection with the other aspects of the disclosure apply respectively.

Also disclosed, but not part of the invention, is a kit comprising a compound of formula (I) obtained or obtainable by a method according to the first aspect of the invention or the compositions comprising such a compound and a container for holding said compound or composition. The kit according to this aspect of the disclosure may further comprise means for administering and/or dispensing the compound or composition to the affected tissue related to the eye and/or the skin and/or the nose and/or the ear and/or the lung of the subject.

Furthermore, the kit according to this aspect of the disclosure may further comprise instructions for use of the container, for example, for dropwise topical administration of the compound or composition to a tissue, specifically to the surface of the eye and/or the skin of a patient or for inhalation. The instructions or directions for use preferably comprised by the kit according to this aspect of the disclosure may be in in any form suited to instruct the user how to perform the administration to the affected tissue of the patient or subject. It may be in any readable or tangible form, preferably in printed form or in any machine- or computer-readable form, for example in form of a machine-readable optical label such as, for example, a barcode or a QR-code. In a particularly preferred embodiment the directions for use are provided in form of an instruction leaflet, product or package insert or as an enclosed label.

### DESCRIPTION OF THE DRAWINGS

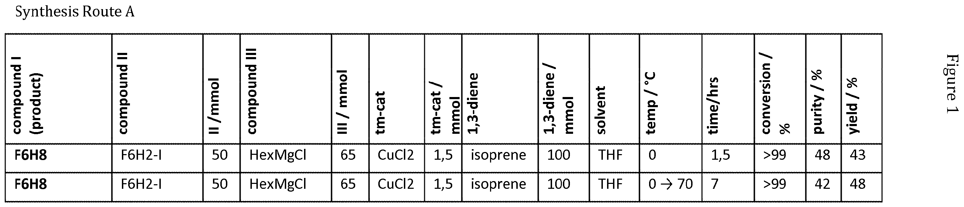

Fig. 1 shows the results of further experiments conducted according to the General Reaction Procedure A as described in example 1A in summarized form.

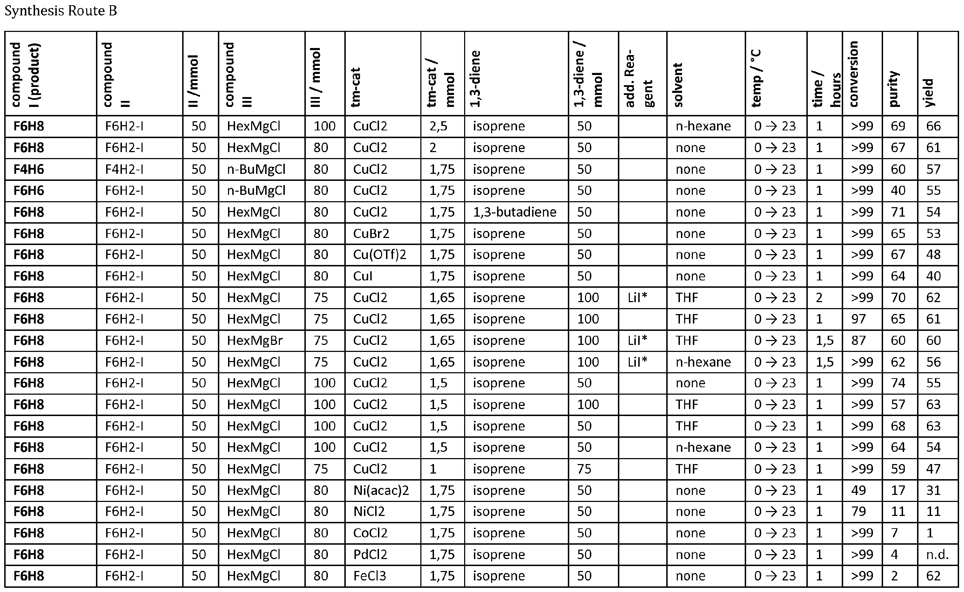

Fig. 2 shows the results of further experiments conducted according to the General Reaction Procedure B as described in example 1B in summarized form.

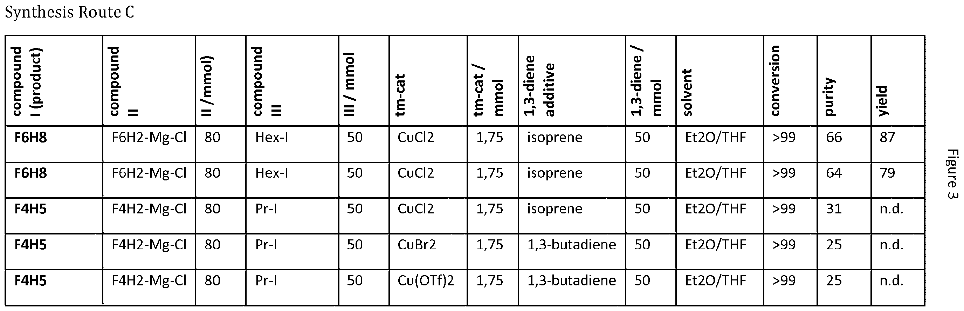

Fig. 3 shows the results of further experiments conducted according to the General Reaction Procedure C as described in example 1C in summarized form.

The following examples serve to illustrate the invention however these are not to be understood as restricting the scope of the invention in any way.

### EXAMPLES

### Example 1: General reaction procedures:

### Example 1A (Reference Example, not according to the invention):

General Reaction Procedure A: Addition of a Grignard compound Ro-Y (III) to a (perfluoro) alkyl halogenide CF₃(CF₂)ₙ(CH₂)ₘ-X (II) as exemplified by the reaction of hexyl magnesium chloride (HexMgCl (IIIc)) to a mixture of F6H2I (F(CF₂)₆(CH₂)₂-I) of formula (IIc) in the presence of isoprene and CuCl₂:

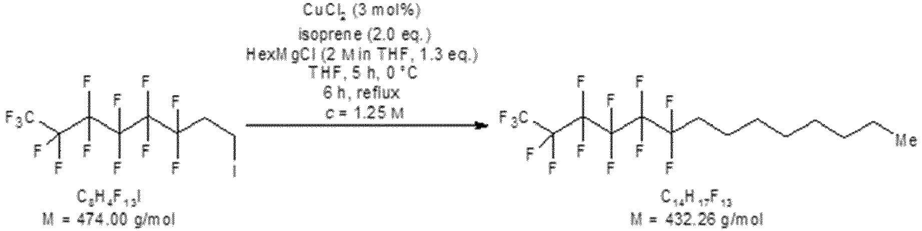

To an ice-cold solution of the 12.3 ml 2-(perfluorohexyl)ethyl iodide (50 mmol; 1 eq.), 10 ml isoprene (100 mmol; 2 eq.) and 0.202 g CuCl₂ (1.5 mmol; 0.03 eq.) in 40 ml anhydrous THF, 32.5 ml of a 2 M hexylmagnesium chloride (65 mmol; 1.3 eq.) solution in THF was added dropwise within 2 h. Then the mixture was refluxed for 5 h. After the addition of aqueous hydrochloric acid (1 M, 50 ml) the phases were separated and the aqueous phase extracted with Et₂O (3 x 50 ml). The combined organic extracts were dried over MgSO₄, filtered and evaporated to dryness to give 24.7 g of yellow liquid as the crude product. GC-analysis showed 42% 1-(perfluorohexyl)octane (yield: 10.4 g, 47.9%).

Following the procedure described above (Procedure A), the products listed in Figure 1 were obtained. In Fig. 1 the following abbreviations and definitions apply:
Compound I, II, III = same meaning as in formulas I, II, III; tm-cat = transition metal catalyst; 1,3-diene = 1,3-diene compound; add. reagent = additional reagent present in the reaction; temp = temperature; time = reaction time; F6H8 = 1-perfluorohexyl-octane; F6H2-I = 1-iodo-2-(perfluorohexyl)-ethane; Hex-Mg-Cl = hexylmagnesium chloride, provided as 2.0 M in THF;

The term "conversion" as used herein refers to the ratio of the compound produced (product) to the starting material (educt) and it is a measure for the extent to which the educt has reacted. In other words, conversion refers to how much reactant was used up as a fraction or percentage of the theoretical amount;

The term "purity" as used herein refers to the purity, in the form of the fraction of product compared to other compounds, as measured by gas chromatography analysis. The ideal value is 100%;

The term "yield" as used herein refers to the amount of product obtained from the chemical reaction. The yields as given here are to be understood as theoretical maximum yields under the assumption that during purification no product is lost. The yields as summarized here are calculated by multiplication of the amount of crude product obtained with the purity as determined by gas chromatography (GC-Yield). The amount of product received divided by the maximum yield multiplied with 100 is the yield in %.

### Example 1B (Reference Example, not according to the invention):

General Reaction Procedure B: Addition of a (perfluoro) alkyl halogenide CF₃(CF₂)ₙ(CH₂)ₘ-X (II) to a precooled Grignard compound Ro-Y (III) as exemplified by the addition of 2-(perfluorohexyl)ethyl iodide (F6H2I (IIc)) to precooled solution of hexylmagnesium chloride of formula (IIIc):

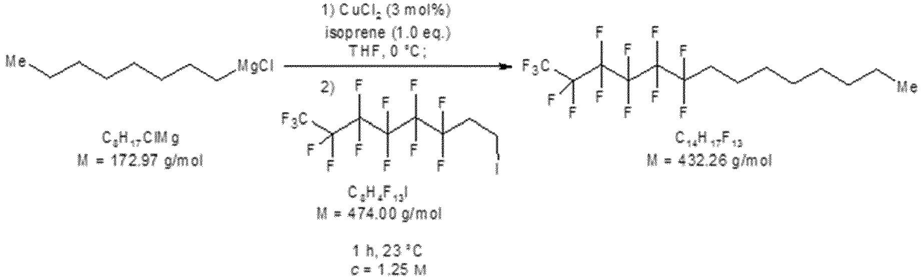

To an ice-cold cooled solution of 50 ml of a 2M hexyl magnesium chloride (100 mmol; 2 eq.) in 40 ml anhydrous THF, 5.02 ml isoprene (50 mmol; 1 eq.) and 0.202 g CuCl₂ (1.5 mmol; 0.03 eq.) were added, followed by the dropwise addition of 12.3 ml 2-(perfluorohexyl)ethyl iodide (40 mmol; 1 eq.) in anhydrous THF (10 ml) over a period of 12 min. The cooling bath was removed and the mixture stirred for 1 h at room temperature. The reaction mixture was quenched by the addition of aqueous HCl (1 M, 50 ml) at 0 °C. The phases were separated and the aqueous layer extracted with Et₂O (3 x 50 ml). The combined organic phases were dried over MgSO₄, filtered and evaporated to dryness to give 17.15 g of yellow liquid containing 1-(perfluorohexyl)octane and dodecane. The GC analysis showed 68.5% 1-(perfluorohexyl)octane (yield: 11.75 g, 54.4%).

Following the procedure described above (Procedure B), the products listed in Figure 2 were obtained. In Fig. 2 the following definitions and abbreviations apply (in addition to the definitions and abbreviations for Fig. 1):
LiI^{∗}: additional reagent: LiI (2 mmol, 0.04 eq. with regard to compound (II))
F4H2-I = 1-iodo-2-(perfluorobutyl)-ethane;
n-PrMgCl = propylmagnesium chloride, provided as 2.0 M in THF;
n-BuMgCl = n-butylmagnesium chloride provided as 2.0 M in THF;
Hex-I = 1-iodo-hexane;
Pr-I = 1-iodo-propane;
0 → 50: addition of reagent carried out at 0°C, then reaction heated to 50°C;
0 → 70: addition of reagent carried out at 0°C, then reaction heated to 70°C;
0 → 23: addition of reagent carried out at 0°C, then reaction brought to room temperature (23°C);
Solvent: "none" = no further solvent employed besides the solvent the Grignard reagent is provided in;
Et2O/THF = solvent = mixture of diethylether and THF;
Cu(OTf)2 = Copper(II) trifluoromethanesulfonate;
Ni(acac)2 = nickel(II) acetylacetonate.

### Example 1C:

General Reaction Procedure C: Addition of an alkyl halogenide Rₒ-Y to an in situ prepared Grignard compound CF₃(CF₂)ₙ(CH₂)ₘ-X (II) as exemplified by the addition of 1-iodohexane to 2-(perfluorohexyl)ethyl magnesium chloride prepared in situ:

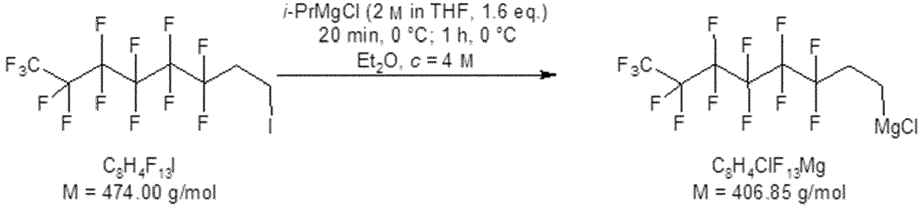

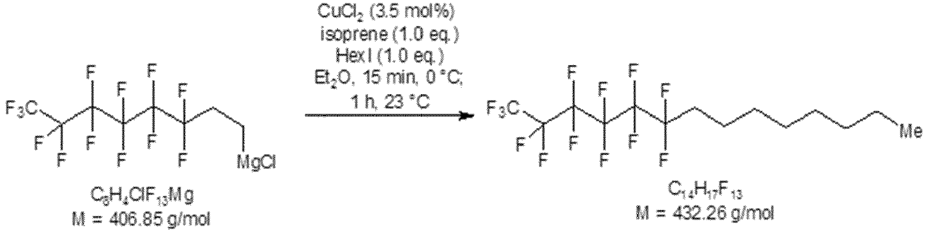

A solution of 19.6 ml 2-(perfluorohexyl)ethyl iodide (80 mmol; 1.6 eq.) in 20 ml anhydrous diethylether (Et₂O) was cooled to 2 °C and 40 ml of a 2M solution of *i-*PrMgCl (*iso*-propylmagnesium chloride; 80 mmol; 1.6 eq.) in anhydrous THF was added within 20 minutes. Then, the mixture comprising the *in situ* generated 2-(perfluorohexyl) ethyl magnesium chloride was stirred for 1 h at 0 °C. Then, 5.02 ml isoprene (50 mmol; 1.0 eq.) and 0.23 g CuCl₂ (1.75 mmol; 0.035 eq.) were added to the mixture. Afterwards a solution of Hex-I (1-iodohexane; 50 mmol; 1 eq.) in anhydrous Et₂O (20 ml) was introduced over a period of 15 minutes. After complete addition, the reaction mixture was stirred for 1 h at room temperature. Then, the product was extracted with hexanes (40-60°C, 3 x 50 ml). The combined organic phases were washed with saturated aqueous Na₂S₂O₃-solution (1 x 50 ml), dried over MgSO₄ and activated charcoal, filtered over Celite^{®} and evaporated to dryness to give 28.56 g of orange liquid as crude product. GC-analysis showed 65.7% 1-(perfluorohexyl) octane (yield: 18.76 g, 86.8%).

Following the procedure described above (Procedure C), the products listed in Figure 3 were obtained. In Fig. 3 the following definitions and abbreviations apply (in addition to those of Figs. 1 and 2):
F6H2-Mg-Cl = 2-(perfluorohexyl)ethylmagnesium chloride; in situ generated by transmetallation from 1-iodo-2-(perfluorohexyl)-ethane and iso-propylmagnesium chloride:
F4H2-Mg-Cl = 2-(perfluorobutyl)ethylmagnesium chloride prepared by transmetallation from 1-iodo-2-(perfluorobutyl)-ethane and isopropylmagnesium bromide

Example 1D (Reference Example, not according to the invention): Performed according to General Reaction Procedure A: Addition of a Grignard compound Ro-Y (III) to a (perfluoro) alkyl halogenide CF₃(CF₂)ₙ(CH₂)ₘ-X (II) as exemplified by the reaction of n-propyl magnesium chloride (nPrMgCl (IIIc)) to a mixture of F4H2I (F(CF₂)₄(CH₂)₂-I) of formula (IIc) in the presence of isoprene and CuCl₂:

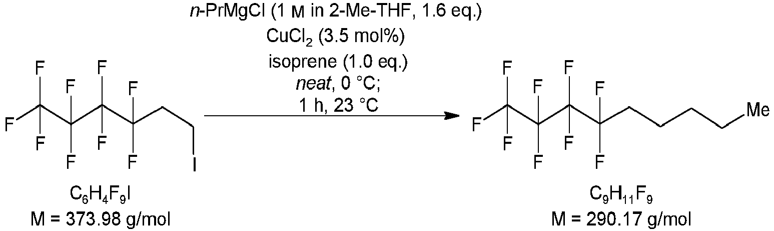

At 7 °C isoprene (8.53 g, 125 mmol, 12.5 ml, 1.00 eq.) and CuCl₂ (0.588 g, 4.38 mmol, 0.035 eq.) was added to *n*-PrMgCl (1 M in 2-Me-THF, 200 mmol, 200 ml, 1.6 eq.). Then, 2-(perfluorobutyl)ethyl iodide (46.7 g, 125 mmol, 24.1 ml, 1.00 eq.) was added dropwise over a period of 21 minutes. After complete addition, the cooling bath was removed, and the mixture stirred for 1 h at room temperature. Then, the reaction mixture was cooled and aqueous NH₄Cl-solution (1 M, 100 ml) was added dropwise followed by stirring for 1 h at room temperature. The mixture was then transferred into a separation funnel and the two phases were separated. The organic phase was washed with aqueous Na₂S₂O₃-solution (1 x 100 ml and 1 x 50 ml) and water (5 x 200 ml). The remaining organic phase was dissolved in EtOH (100 ml) and water (250 ml) was added. A lower phase appeared. This procedure was repeated once more. In the end, the lower phase was collected, dried over MgSO₄ and filtered to give 13.42 g of clear yellowish liquid. The crude product was analyzed by GC. This analysis showed 68.5% 1-(perfluorobutyl)pentane (yield: 9.19 g, 25.3%).

### Example 2: Purities of crude product: Comparison to conventional SFA synthesis routes

### Example 2A: Purity of F4H5

The purities of F4H5 (compound la, perfluoro-butyl-pentane) obtained as the crude product when synthesized according to the method of present invention (Grignard route; reaction of 1-iodo-2-(perfluorobutyl)-ethane with n-propylmagnesium chloride or alternatively by reaction of 2-(perfluorobutyl)ethylmagnesium chloride with 1-iodo-propane) was compared to conventional 2-step synthesis starting with a radical addition followed reductive dehalogenation by zinc/gaseous HCl (Zn/HCl) or catalytic hydrogenation employing a palladium on charcoal (Pd/C) as the catalyst. Herein, the content of the desired product (Ia) in comparison to the very difficult to separate side products IVa and Va as determined by gas chromatography are shown: The results are summarized in Table 2. The values given in % refer to relative amounts of the respective compounds in the crude product mixtures as determined by GC-analysis.

**Table 2:**

| **Compound** | **Compound, formula** | **Zn/HCl** | **Pd/C** | **Grignard** |
|---|---|---|---|---|
| Ia | F(CF₂)₄-(CH₂)₅H | 57-76% | 79-86% | 31% |
| IVa | F(CF₂)₄-(CH(CH3) (CH₂)₃H | 1.4-2.5% | 3-10% | 0% |
| Va | F(CF₂)₄-CH=CH-(CH₂)₃H | 3.5-5.4% | 0.5-3.0 % | 0% |

It was shown that the synthesis route according to the present invention (Grignard route) does not produce the isomeric side product IVa and does not produce the olefinic side product Va.

### Example 2B: Purity F6H8

The purities of the crude product of F6H8 (compound (Ib),1-perfluorohexyl-octane) synthesized according to the present invention (Grignard route) was compared to conventional 2-step synthesis, starting with a radical addition followed reductive dehalogenation by Zinc/gaseous HCl (Zn/HCl) or catalytic hydrogenation employing a palladium on charcoal (Pd/C) as catalyst. Herein, the content of the desired product (Ib) in comparison to the very difficult to separate side products (IVb) and (Vb) as determined by gas chromatography are shown. The results are summarized in Table 3. The values given in % refer to relative amounts of the respective compounds in the crude product mixtures as determined by GC-analysis.

**Table 3:**

| **Compound** | **Compound, formula** | **Zn/HCl** | **Pd/C** | **Grignard** |
|---|---|---|---|---|
| Ib | F(CF₂)₆-(CH₂)₈H | 86% | 69-84% | 60-74 % |
| IVb | F(CF₂)₆-(CH(CH3) (CH₂)₆H | 0.9% | 2.6-3,0 % | 0% |
| Vb | F(CF₂)₆-CH=CH-(CH₂)₆H | 0.01% | 0.4-0.7 % | 0% |

It was shown that the synthesis route according to the present invention (Grignard route) does not produce the isomeric side product IVb and does not produce the olefinic side product Vb.

## Claims

1. A method for preparing a compound of formula (I)
F-(CF₂)ₙ-(CH₂)ₘ-Rₒ (I),
wherein
n is an integer from 2 to 12,
m is 2,
Rₒ is a linear or branched saturated alkyl and o depicts the number of carbon atoms,
o is an integer from 1 to 12, and wherein
m+o is an integer from 3 to 12;
comprising reacting a fluorinated compound of formula (II)
F-(CF₂)ₙ-(CH₂)ₘ-X (II),
wherein
X MgCl, MgBr, or MgI, and
n and m are as defined in formula (I),
with a non-fluorinated compound of formula (III)
Rₒ-Y (III),
wherein
Y is Cl, Br, I, and
Rₒ is as defined in formula (I).

2. The method according to claim 1, wherein the compound of formula (II) is prepared in situ.

3. The method according to claim 1 or 2, wherein the compound of formula (II) is formed by transfer of the magnesium halide substituent of a further Grignard reagent to the halogenated precursor of the compound of formula (II) with X= Cl, Br or I.

4. The method according to any preceding claim, wherein the compound of formula (II) is added in form of their halogenated precursors ,followed by treatment with a further Grignard reagent selected from the group consisting of isopropylmagnesium chloride, isopropylmagnesium bromide, ethylmagnesium chloride, ethylmagnesium bromide, phenylmagnesium chloride, phenylmagnesium bromide, preferably selected from the group consisting of isopropylmagnesium chloride, isopropylmagnesium bromide, ethylmagnesium chloride, ethylmagnesium bromide, thereby generating the compound of formula (II) in situ.

5. The method according to claim 4, wherein the further Grignard reagent is selected from the group consisting of isopropylmagnesium chloride, isopropylmagnesium bromide, ethylmagnesium chloride, ethylmagnesium bromide.

6. The method according to claims 1 to 5, wherein the reaction is carried out in presence of a transition metal compound.

7. The method according to claim 6, wherein the transition metal compound is a copper compound.

8. The method according to any of the claims 1 to 7, wherein the reaction is carried out in the presence of a 1,3-diene compound.

9. The method according to claim 8, wherein the 1,3-diene compound is isoprene.

10. The method according to any of claims 1 to 9, wherein
Rₒ is a linear saturated alkyl group.

11. The method according to any one of claims 1 to 10, wherein
n is 4, 5 or 6,
m is 2,
o is an integer from 1 to 8, and
m+o is an integer of 4 to 8.

12. The method according to any one of claims 1 to 10, wherein
n is 4,
m is 2, and
o is 3.

13. The method according to any one of claims 1 to 10, wherein
n is 6,
m is 2, and
o is 6.

14. The method according to any preceding claim, wherein
X is is MgCl, MgBr or MgI, and
Y is I.

## Patentansprüche

1. Verfahren zur Herstellung einer Verbindung der Formel (I)
F-(CF₂)ₙ-(CH₂)ₘ-Rₒ (I),
wobei
n für eine ganze Zahl von 2 bis 12 steht,
m für 2 steht,
Rₒ für ein lineares oder verzweigtes gesättigtes Alkyl steht und o für die Zahl von Kohlenstoffatomen steht,
o für eine ganze Zahl von 1 bis 12 steht und wobei
m + o für eine ganze Zahl von 3 bis 12 steht;
umfassend das Umsetzen einer fluorierten Verbindung der Formel (II)
F-(CF₂)ₙ-(CH₂)ₘ-X (II),
wobei
X für MgCl, MgBr oder MgI steht und
n und m wie in Formel (I) definiert sind,
mit einer nichtfluorierten Verbindung der Formel (III)
Rₒ-Y (III),
wobei
Y für Cl, Br oder I steht und
Rₒ wie in Formel (I) definiert ist.

2. Verfahren nach Anspruch 1, wobei die Verbindung der Formel (II) in situ hergestellt wird.

3. Verfahren nach Anspruch 1 oder 2, wobei die Verbindung der Formel (II) durch Übertragung des Magnesiumhalogenid-Substituenten eines weiteren Grignard-Reagenzes auf die halogenierte Vorstufe der Verbindung der Formel (II) mit X = Cl, Br oder I gebildet wird.

4. Verfahren nach einem der vorhergehenden Ansprüche, wobei die Verbindung der Formel (II) in Form ihrer halogenierten Vorstufen zugegeben wird und anschließend mit einem weiteren Grignard-Reagenz, das aus der Gruppe bestehend aus Isopropylmagnesiumchlorid, Isopropylmagnesiumbromid, Ethylmagnesiumchlorid, Ethylmagnesiumbromid, Phenylmagnesiumchlorid und Phenylmagnesiumbromid ausgewählt wird und vorzugsweise aus der Gruppe bestehend aus Isopropylmagnesiumchlorid, Isopropylmagnesiumbromid, Ethylmagnesiumchlorid und Ethylmagnesiumbromid ausgewählt wird, behandelt wird, wodurch die Verbindung der Formel (II) in situ gebildet wird.

5. Verfahren nach Anspruch 4, wobei das weitere Grignard-Reagenz aus der Gruppe bestehend aus Isopropylmagnesiumchlorid, Isopropylmagnesiumbromid, Ethylmagnesiumchlorid und Ethylmagnesiumbromid ausgewählt wird.

6. Verfahren nach den Ansprüchen 1 bis 5, wobei die Umsetzung in Gegenwart einer Übergangsmetallverbindung durchgeführt wird.

7. Verfahren nach Anspruch 6, wobei es sich bei der Übergangsmetallverbindung um eine Kupferverbindung handelt.

8. Verfahren nach einem der Ansprüche 1 bis 7, wobei die Umsetzung in Gegenwart einer 1,3-Dienverbindung durchgeführt wird.

9. Verfahren nach Anspruch 8, wobei es sich bei der 1,3-Dienverbindung um Isopren handelt.

10. Verfahren nach einem der Ansprüche 1 bis 9, wobei
Rₒ für eine lineare gesättigte Alkylgruppe steht.

11. Verfahren nach einem der Ansprüche 1 bis 10, wobei
n für 4, 5 oder 6 steht,
m für 2 steht,
o für eine ganze Zahl von 1 bis 8 steht und
m + o für eine ganze Zahl von 4 bis 8 steht.

12. Verfahren nach einem der Ansprüche 1 bis 10, wobei
n für 4 steht,
m für 2 steht und
o für 3 steht.

13. Verfahren nach einem der Ansprüche 1 bis 10, wobei
n für 6 steht,
m für 2 steht und
o für 6 steht.

14. Verfahren nach einem der vorhergehenden Ansprüche, wobei
X für MgCl, MgBr oder MgI steht und
Y für I steht.

## Revendications

1. Procédé de préparation d'un composé de formule (I)
F-(CF₂)ₙ-(CH₂)ₘ-Rₒ (I),
n étant un entier de 2 à 12,
m valant 2,
Rₒ étant un alkyle saturé linéaire ou ramifié et o représentant le nombre d'atomes de carbone,
0 étant un nombre entier de 1 à 12, et
m+o étant un nombre entier de 3 à 12 ;
ledit procédé comprenant la réaction d'un composé fluoré de formule (II)
F-(CF₂)ₙ-(CH₂)ₘ-X (II),
X étant MgCl, MgBr ou MgI, et
n et m étant tels que définis dans la formule (I),
avec un composé non fluoré de formule (III)
Rₒ-Y (III),
Y étant Cl, Br, I, et
Rₒ étant tel que défini dans la formule (I).

2. Procédé selon la revendication 1, le composé de formule (II) étant préparé in situ.

3. Procédé selon la revendication 1 ou 2, le composé de formule (II) étant formé par transfert du substituant halogénure de magnésium d'un autre réactif de Grignard sur le précurseur halogéné du composé de formule (II) avec X = Cl, Br ou I.

4. Procédé selon l'une quelconque des revendications précédentes, le composé de formule (II) étant ajouté sous la forme de ses précurseurs halogénés, puis un traitement étant effectué avec un autre réactif de Grignard choisi dans le groupe comprenant le chlorure d'isopropylmagnésium, le bromure d'isopropylmagnésium, le chlorure d'éthylmagnésium, le bromure d'éthylmagnésium, le chlorure de phénylmagnésium, le bromure de phénylmagnésium, de préférence choisis dans le groupe comprenant le chlorure d'isopropylmagnésium, le bromure d'isopropylmagnésium, le chlorure d'éthylmagnésium, le bromure d'éthylmagnésium, de manière à générer le composé de formule (II) in situ.

5. Procédé selon la revendication 4, l'autre réactif de Grignard étant choisi dans le groupe comprenant le chlorure d'isopropylmagnésium, le bromure d'isopropylmagnésium, le chlorure d'éthylmagnésium, le bromure d'éthylmagnésium.

6. Procédé selon les revendications 1 à 5, la réaction étant effectuée en présence d'un composé de métal de transition.

7. Procédé selon la revendication 6, le composé métal de transition étant un composé cuivre.

8. Procédé selon l'une quelconque des revendications 1 à 7, la réaction étant effectuée en présence d'un composé 1,3-diène.

9. Procédé selon la revendication 8, le composé 1,3-diène étant l'isoprène.

10. Procédé selon l'une quelconque des revendications 1 à 9, Rₒ étant un groupe alkyle saturé linéaire.

11. Procédé selon l'une quelconque des revendications 1 à 10,
n valant 4, 5 ou 6,
m valant 2,
o étant un entier de 1 à 8, et
m+o étant un entier de 4 à 8.

12. Procédé selon l'une quelconque des revendications 1 à 10,
n valant 4,
m valant 2 et
o valant 3.

13. Procédé selon l'une des revendications 1 à 10,
n valant 6,
m valant 2 et
o valant 6.

14. Procédé selon l'une quelconque des revendications précédentes,
X étant MgCl, MgBr ou MgI, et
Y étant 1.
